(19) Europäisches Patentamt
European Patent Office
Office européen des brevets

(11) **EP 3 769 662 B1**

(12) **EUROPÄISCHE PATENTSCHRIFT**

(45) Veröffentlichungstag und Bekanntmachung des Hinweises auf die Patenterteilung:
**05.04.2023 Patentblatt 2023/14**

(21) Anmeldenummer: **20187842.8**

(22) Anmeldetag: **27.07.2020**

(51) Internationale Patentklassifikation (IPC):
**A61B 1/00** *(2006.01)* **G02B 6/44** *(2006.01)*

(52) Gemeinsame Patentklassifikation (CPC):
**G02B 6/4416; A61B 1/0017;** A61B 1/24; H01B 11/22

(54) **OPTISCH-ELEKTRISCHES LEITERSYSTEM MIT ADAPTERHÜLSE**

OPTO-ELECTRICAL CONDUCTOR SYSTEM WITH ADAPTER SLEEVE

SYSTÈME DE CONDUCTEUR OPTIQUE-ÉLECTRIQUE POURVU DE MANCHON ADAPTATEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.07.2019 DE 102019120324**

(43) Veröffentlichungstag der Anmeldung:
**27.01.2021 Patentblatt 2021/04**

(73) Patentinhaber: **SCHOTT AG**
**55122 Mainz (DE)**

(72) Erfinder:
• **SCHULTHEIS, Bernd**
**55270 Schwabenheim (DE)**
• **KEIPER, Oliver**
**65510 Hünstetten (DE)**
• **HENN, Christian**
**55546 Frei-Laubersheim (DE)**

(74) Vertreter: **Blumbach · Zinngrebe Patentanwälte PartG mbB**
**Alexandrastraße 5**
**65187 Wiesbaden (DE)**

(56) Entgegenhaltungen:
**WO-A1-94/22039** **CN-B- 104 155 716**
**JP-A- H0 819 557** **US-A- 5 497 442**
**US-A1- 2010 057 179** **US-A1- 2019 090 998**

## Beschreibung

Die Erfindung betrifft ein optisch-elektrisches Leitersystem mit einem organisch ummantelten und leitfähig beschichteten Lichtwellenleiter welches in eine Anschlussfassung eines handhaltbaren Geräts einsetzbar ist.

Lichtwellenleiter dienen zur Übertragung von Licht oder Energie für zahlreiche Anwendungen. Dabei ist es mitunter wünschenswert, zusätzlich zur Lichtübertragung eine Übertragung von elektrischen Signalen zu ermöglichen, beispielsweise, um die Lichtübertragung in Abhängigkeit von den elektrischen Signalen zu steuern. Dies kann durch eine auf dem Lichtwellenleiter bzw. auf dessen Ummantelung aufgebrachte Leitschicht ermöglicht werden.

Die deutsche Patentanmeldung DE 10 2012 109 088 A1 beschreibt etwa ein faseroptisches Konversionsmodul als Teil einer Beleuchtungseinrichtung bei einem Fahrzeug, wobei Lichtleitfasern mit elektrisch leitfähiger Beschichtung zum Einsatz kommen können, welche es erlauben, einen Bruch von Lichtleitfasern festzustellen. Ein Bruch einer Lichtleitfaser führt zu einer Unterbrechung eines elektrischen Stromkreispfades, was durch fehlenden oder abfallenden Strom in Nachweismitteln feststellbar ist und zur Abschaltung von Anregungslichtquellen ausgenutzt wird.

Die deutsche Patentanmeldung DE 10 2006 029 203 A1 betrifft eine Licht emittierende Vorrichtung mit einem Lichtleiter. Auf der Oberfläche des Mantelbereichs des Lichtleiters kann eine elektrisch leitfähige Verbindung vorhanden sein, die sich um den Mantelbereich windet bzw. umlaufend um den Lichtleiter angeordnet ist und somit eine mögliche Beschädigung bzw. einen Bruch des Lichtleiters an verschiedenen Stellen detektieren kann.

Das US-Patent 5 135 295 beschreibt piezoelektrische und Ultraschallvorrichtungen, die optische Fasern verwenden, die mit dünnen piezoelektrischen und ferroelektrischen Blei-Zirkonat-Titanat-Filmen beschichtet sind. Die PZT-Dünnschichten werden chemisch durch ein Sol-Gel-Verfahren hergestellt.

Unabhängig von einer leitfähigen Beschichtung kommen Lichtwellenleiter allgemein auch im Bereich der Medizintechnik zum Einsatz. Die WO2019057316A1 bzw. US2019090998A1 betreffen beispielsweise ein Verfahren und eine Anordnung zum Reinigen eines umfänglich geschlossenen Kanals mittels eines einen Laserstrahl führenden Lichtleiters.

WO9422039A1 betrifft ein optisches/elektrisches koaxiales Signalkabel umfassend einen faseroptischen Kern, eine Schicht mit leitenden Metallsträngen, die den Kern spiralförmig umgeben, eine Isolationsschicht, die die leitenden Metallstränge umgibt, und eine leitfähige Metallabschirmung, die die Isolationsschicht umgibt.

Wünschenswert wäre es, Lichtwellenleiter, die beispielsweise zur Anbringung an einem handhaltbaren Gerät oder Handstück vorgesehen sind auf einfache Weise modular und austauschbar befestigen zu können, wobei gleichzeitig eine präzise Anordnung und, im Falle einer leitfähigen Beschichtung, eine elektrische Kontaktierung ermöglicht wird.

Die Aufgabe der Erfindung wird durch den Gegenstand der unabhängigen Ansprüche gelöst. Vorteilhafte Weiterbildungen der Erfindung sind in den Unteransprüchen definiert.

Die Erfindung betrifft ein optisch-elektrisches Leitersystem mit einer optisch-elektrischen Leiteranordnung und einer Adapterhülse zur Aufnahme der optisch-elektrische Leiteranordnung.

Die optisch-elektrische Leiteranordnung umfasst einen Lichtwellenleiter mit einer äußeren, organischen Mantelschicht, und eine unmittelbar oder mittelbar auf der äußeren Mantelschicht des Lichtwellenleiters aufgebrachte Leitschicht bestehend aus einer einzelnen Schicht oder einer Abfolge von Schichten.

Die Adapterhülse ist zumindest bereichsweise elektrisch leitfähig ausgebildet und dient einerseits dazu, die optisch-elektrische Leiteranordnung mechanisch zu erfassen, und andererseits dazu, deren Leitschicht elektrisch zu kontaktieren, und zwar insbesondere derart, dass das optisch-elektrische Leitersystem zusammen mit der umgebenden Adapterhülse in eine, z.B. an einem handhaltbaren Gerät oder Handstück angeordnete, Anschlussfassung zur Übertragung optischer und/oder elektrischer Signale einsetzbar ist.

In geometrischer Hinsicht weist die Adapterhülse vorzugsweise einen äußeren Hülsenkörper auf welcher eine innere Aufnahmeöffnung umschließt. Die optisch-elektrische Leiteranordnung kann sich dann entsprechend entlang einer durch die Adapterhülse definierten Hülsenachse durch die innere Aufnahmeöffnung der Adapterhülse erstrecken.

Vorzugsweise ist die innere Aufnahmeöffnung der Adapterhülse zylindrisch geformt, so dass vorzugsweise eine optisch-elektrische Leiteranordnung mit rundem Querschnitt darin passend aufgenommen werden kann. Ferner ist die innere Aufnahmeöffnung der Adapterhülse bevorzugt konzentrisch zum äußeren Hülsenkörper angeordnet, wobei dieser ggf. ebenfalls zylindrisch ausgebildet sein kann.

Vorzugsweise ist die optisch-elektrische Leiteranordnung mit einem (proximalen) Ende in die Adapterhülse eingeführt, wobei dieses Ende mit der Adapterhülse ggf. bündig abschließen kann. Zumindest mit dem anderen (distalen) Ende ragt die optisch-elektrische Leiteranordnung vorzugsweise aus der Adapterhülse heraus. Da typischerweise Licht am proximalen Ende in den Lichtwellenleiter eingekoppelt wird, welches den Lichtwellenleiter am distalen Ende wieder verlässt, ist die optisch-elektrische Leiteranordnung vorzugsweise derart in der Adapterhülse erfasst, dass zumindest der Lichtwellenleiter von beiden Seiten der Adapterhülse zugänglich ist.

Das distale Ende der optisch-elektrischen Leiteranordnung, das in der Regel zur Auskopplung von Licht dient, kann im Wesentlichen flach ausgebildet sein. Es kann aber auch vorgesehen sein, dass das distale Ende der optisch-elektrischen Leiteranordnung hinsichtlich dessen im Wesentlichen flacher Geometrie modifiziert und/oder strukturiert und/oder aufgeraut, insbesondere zumindest teil- oder abschnittsweise, z.B. kegelstumpfförmig, mit einem Winkel kleiner 90° zur Längsachse der optisch-elektrischen Leiteranordnung ausgebildet ist, bspw. um eine geforderten oder ausgerichtete Abstrahlung des am proximalen Ende eingekoppelten und darin geführten Lichtes modifiziert bereitzustellen. Dies kann bspw. durch Anspitzen, zumindest einseitigem Anschleifen und/oder Polieren oder mittels physikalischer oder chemischer Ätzprozeße erreicht werden.

Um die optisch-elektrische Leiteranordnung mechanisch zu erfassen weist die Adapterhülse bevorzugt einen, insbesondere eigenen, Fixierungsabschnitt auf. In diesem Fixierungsabschnitt kann ein Kleber eingebracht sein, welcher die optisch-elektrische Leiteranordnung zusätzlich fixiert, wobei der Kleber in radialer Richtung insbesondere eine Dicke zwischen 1 und 500 Mikrometer aufweist, vorzugsweise zwischen 5 und 100 Mikrometer aufweist, besonders bevorzugt zwischen 10 und 50 Mikrometer aufweist.

Wenn die Adapterhülse in einer komplementären Anschlussfassung eingesetzt ist, kann Licht über die Anschlussfassung in den Lichtwellenleiter der optisch-elektrischen Leiteranordnung eingekoppelt werden.

Um in diesem Fall eine präzise Einkopplung möglichst zentral in den Lichtwellenleiter zu gewährleisten, kann vorgesehen sein, dass der Fixierungsabschnitt der Adapterhülse die optisch-elektrische Leiteranordnung mit einem radialen Toleranz von weniger als $50\,\mu m$ erfasst, vorzugsweise von weniger als $30\,\mu m$ erfasst.

Dies kann insbesondere ermöglicht werden indem der Fixierungsabschnitt der Adapterhülse gegenüber der optisch-elektrischen Leiteranordnung ein radiales Spiel von weniger als $50\,\mu m$, vorzugsweise von weniger als $30\,\mu m$ aufweist. Auch im Fall, dass eine oder mehrere äußere Schichten der optisch-elektrischen Leiteranordnung abisoliert sind kann ein Spiel von weniger als $50\,\mu m$, vorzugsweise von weniger als $30\,\mu m$, gegenüber der jeweiligen äußersten Komponente der optisch-elektrischen Leiteranordnung vorgesehen sein.

Besonders bevorzugt erfasst der Fixierungsabschnitt der Adapterhülse die optisch-elektrische Leiteranordnung derart, dass der Lichtwellenleiter der optisch-elektrischen Leiteranordnung zentrisch zur Hülsenachse mit einem Konzentrizitätsfehler von weniger als $50\,\mu m$, vorzugsweise von weniger als $30\,\mu m$, erfasst ist.

Erfindungsgemäß umfasst die Adapterhülse einen, insbesondere eigenen, ggf. aber auch gemeinsam mit dem Fixierungsabschnitt ausgebildeten, elektrisch leitfähigen Kontaktierungsabschnitt, welcher die Leitschicht der optisch-elektrischen Leiteranordnung elektrisch kontaktiert. In diesem Kontaktierungsabschnitt ist ein elektrischer Leitkleber eingebracht, welcher die Leitschicht der optisch-elektrischen Leiteranordnung elektrisch kontaktiert, wobei der Leitkleber in radialer Richtung insbesondere eine Dicke zwischen 1 und 500 Mikrometer aufweist, vorzugsweise zwischen 25 und 250 Mikrometer aufweist, besonders bevorzugt zwischen 50 und 150 Mikrometer aufweist.

Es kann vorgesehen sein, dass die innere Aufnahmeöffnung der Adapterhülse im Bereich des Fixierungsabschnitts einen kleineren Querschnitt aufweist als im Bereich des Kontaktierungsabschnitts. In diesem Fall kann zwischen dem Fixierungsabschnitt und dem Kontaktierungsabschnitt einen Übergangsbereich von dem kleineren Querschnitt auf den größeren Querschnitt angeordnet sein, wobei ein solcher Übergangsbereich z.B. als Konus oder als Ringschulter ausgebildet sein kann.

Grundsätzlich kann die Erfindung für optisch-elektrische Leiteranordnungen und Adapterhülsen jeder Größe zum Einsatz kommen. Möglich ist z.B. dass die Adapterhülse einen Außendurchmesser von kleiner als 20 Millimeter, vorzugsweise von kleiner als 10 Millimeter, besonders bevorzugt von kleiner 5 Millimeter aufweist und/oder dass die Adapterhülse einen Innendurchmesser von kleiner als 2000 Mikrometer, vorzugsweise von kleiner als 1000 Mikrometer, besonders bevorzugt von kleiner 500 Mikrometer aufweist.

Da die Adapterhülse zumindest bereichsweise elektrisch leitfähig ausgebildet ist, kann es sich anbieten, dass die Adapterhülse ein Metall, z.B. Edelstahl oder Neusilber, oder einen leitfähigen Kunststoff oder eine leitfähige Keramik umfasst oder daraus besteht.

Eine mögliche Anwendung des vorgestellten optisch-elektrischen Leitersystems liegt in der Messung einer Impedanz zwischen der Adapterhülse, welche insbesondere das proximale Ende der optisch-elektrischen Leiteranordnung elektrisch kontaktiert, und der Leitschicht, insbesondere am distalen Ende der optisch-elektrischen Leiteranordnung, oder einem die Leitschicht umgebenden leitfähigen Medium, in das die optisch-elektrischen Leiteranordnung mit ihrem distalen Ende eingetaucht sein kann.

Insbesondere zu diesem Zweck, aber auch unabhängig davon, kann vorgesehen sein, dass zwischen der Adapterhülse und der Leitschicht der optisch-elektrischen Leiteranordnung eine Impedanz messbar ist, wobei die Impedanz einerseits einen Leitschichtanteil aufweist und andererseits einen Kontaktierungsanteil aufweist.

Der Leitschichtanteil kann dabei von der Impedanz der Leitschicht und/oder der Impedanz der Leitschicht zu einem die optisch-elektrischen Leiteranordnung umgebenden Medium abhängen oder dadurch gebildet sein. Der Kontaktierungsanteil hingegen kann zumindest von dem ohmschen Widerstand des im Kontaktierungsabschnitt eingebrachten Leitklebers und/oder dem kapazitiven Widerstand des im Kontaktierungsabschnitt eingebrachten Leitklebers abhängen oder dadurch gebildet sein.

In einer bevorzugten Ausführungsform ist der Kontaktierungsanteil der Impedanz kleiner gleich dem 10-fachen, vorzugsweise kleiner gleich dem 5-fachen, besonders bevorzugt kleiner gleich dem 0,5-fachen des Leitschichtanteils der Impedanz.

Insbesondere ist die beschriebene zwischen der Adapterhülse und der Leitschicht der optisch-elektrischen Leiteranordnung messbare Impedanz bei einer bestimmten Auswertefrequenz oder über einen Auswertefrequenzbereich messbar, wobei die Auswertefrequenz oder der Auswertefrequenzbereich vorzugsweise im Bereich von 1 Hz bis 10 GHz, bevorzugter im Bereich von 10 Hz bis 2,4 GHz, nochmals bevorzugter im Bereich von 100 Hz bis 1 MHz, besonders bevorzugt im Bereich von 1 kHz bis 100 kHz und ganz besonders bevorzugt im Bereich von 1 KHz bis 10 kHz liegt.

Ferner ist die zwischen der Adapterhülse und der Leitschicht der optisch-elektrischen Leiteranordnung messbare Impedanz bevorzugt zwischen dem äußeren Hülsenkörper der Adapterhülse und einem Bereich der Leitschicht in einem aus der Adapterhülse herausragenden, vorzugsweise endseitigem, Abschnitt der optisch-elektrischen Leiteranordnung messbar.

Wie beschrieben kann der Kontaktierungsanteil der Impedanz zumindest von dem ohmschen bzw. kapazitiven Widerstand des im Kontaktierungsabschnitt eingebrachten Leitklebers abhängen. Ferner kann der Kontaktierungsanteil aber unter anderem auch von dem ohmschen Widerstand des im Fixierungsabschnitt eingebrachten Klebers und/oder dem kapazitiven Widerstand des im Fixierungsabschnitt eingebrachten Klebers abhängen.

Wenn die optisch-elektrischen Leiteranordnung eine Barriereschicht umfasst, kann auch diese zu dem Kontaktierungsanteil der Impedanz beitragen. Eine Barriereschicht kann dazu dienen, ein Eindiffundieren von Sauerstoff und/oder Ionen von sauren oder alkalischen Lösungen in die Leitschicht zu hemmen und kann z.B. unmittelbar oder mittelbar auf der Leitschicht aufgebracht sein und/oder eine oberflächliche Schicht der Leitschicht mit zumindest einer veränderten Materialeigenschaft umfassen oder als solche ausgebildet sein. Eine Barriereschicht kann z.B. auch als natürliche Oxidationsschicht der Leitschicht (z.B. bei einer Aluminium-Leitschicht), und/oder als Kontaminationsfilm (Feuchtebelag, Partikel-Kontamination und/oder Schmutz) auf der Leitschicht ausgebildet sein. Auch hier können relativ hochohmige Übergangswiderstände resultieren, ohne dass eine separate Barriereschicht aufgebracht wurde.

In Fall einer Barriereschicht kann der Kontaktierungsanteil ferner abhängig sein von dem ohmschen Widerstand der Barriereschicht im Bereich des im Fixierungsabschnitt eingebrachten Klebers und/oder dem kapazitiven Widerstand der Barriereschicht im Bereich des im Fixierungsabschnitt eingebrachten Klebers.

Weiterhin kann vorgesehen sein, dass der ohmsche Widerstand der Adapterhülse zwischen 1 und 1000 Milliohm liegt, vorzugsweise zwischen 10 und 1000 Milliohm liegt, besonders bevorzugt zwischen 10 und 100 Milliohm liegt. Je nach Art der Kontaktierung und Bauform der Adapterhülse kann dies insbesondere der ohmsche Widerstand in radialer Richtung durch den äußeren Hülsenkörper sein.

Ferner kann vorgesehen sein, dass der ohmsche Widerstand der Leitschicht zwischen 1 und 1000 Ohm liegt, vorzugsweise zwischen 10 und 500 Ohm liegt, besonders bevorzugt zwischen 10 und 100 Ohm liegt. Dies kann insbesondere der ohmsche Widerstand entlang der Längsausdehnung der optisch-elektrischen Leiteranordnung sein.

Die vorstehenden elektrischen Eigenschaften des optisch-elektrischen Leitersystems können in vorteilhafter Weise dazu dienen, die Messung einer Impedanz oder eine Impedanzänderung beim Eintauchen der optisch-elektrischen Leiteranordnung in ein Medium zu begünstigen.

Gemäß einer Ausführungsform kann z.B. der Impedanzanteil der Kontaktierung der Leitschicht, welcher durch kapazitive und ohmsche Widerstände des Klebers und/oder des elektrischen Leitklebers sowie ggf. einer Barriereschicht bei einer bestimmten Auswertefrequenz gebildet sind, maximal das 10-fache, bevorzugt maximal das 5-fache, besonders bevorzugt maximal das 0,5-fache der Impedanz der Leitschicht und/oder der Impedanz der Leitschicht zu einem das Leitersystem umgebendes leitfähiges Medium sein.

Die Auswertefrequenz kann dabei im Bereich von 100 Hz bis 1 MHz, bevorzugt im Bereich im Bereich von 1 kHz bis 100 kHz, und besonders bevorzugt im Bereich von 1 KHz bis 10 kHz liegen. Höhere Frequenzen können in manchen Fällen weniger geeignet sein, da ggf. bereits im RF-Bereich HF-Störungen auftreten können. Andererseits können in manchen Fällen aus zu niedrigen Frequenzen bei den kapazitiven Übergängen zu große Impedanzen resultieren.

Nachfolgend soll noch näher auf die (in der Adapterhülse aufgenommene) optisch-elektrische Leiteranordnung eingegangen werden.

Wie weiter oben ausgeführt umfasst die optisch-elektrische Leiteranordnung einen Lichtwellenleiter mit einer äußeren, organischen Mantelschicht. Auf der äußeren Mantelschicht kann ein Funktionsschichtsystem angeordnet sein, welches einen Grundschichtbereich umfassen kann, wobei die elektrisch leitende Leitschicht auf dem Grundschichtbereich aufgebracht sein kann. Der Grundschichtbereich kann aus einer einzelnen Schicht oder aus einer Abfolge von Schichten bestehen. Ebenso kann die elektrisch leitende Leitschicht aus einer einzelnen Schicht oder einer Schichtabfolge bestehen.

Der Lichtwellenleiter auf dessen äußerer Mantelschicht sich die Leitschicht oder ggf. ein Funktionsschichtsystem befindet, kann in unterschiedlicher Art ausgebildet sein, wie dem Fachmann bekannt ist. Typischerweise umfasst der Lichtwellenleiter zumindest einen optisch leitenden Kern, vorzugsweise aus Glas, insbesondere Quarzglas, in welchen elektromagnetische Strahlung, beispielsweise die eines Lasers, eingekoppelt werden kann. Der Kern kann unmittelbar umgeben sein von einer Umhüllung, welche sich zwischen dem Kern und der äußeren Mantelschicht befindet. Eine solche Umhüllung kann ebenfalls aus Quarzglas sein. Der innere Kern weist dabei einen größeren Brechungsindex auf als die Umhüllung. Der Kern weist vorzugsweise einen Durchmesser von 10 bis 600 μm auf, die Umhüllung hat typischerweise einen Durchmesser, der dem 1,1- bis 1,5-fachen des Kerndurchmessers entspricht, die Wandstäre der Mantelschicht liegt bevorzugt im Bereich von 1 bis 100 μm. Es kann auch vorgesehen sein, dass der innere Kern unmittelbar von der äußeren Mantelschicht umgeben ist. In diesem Fall ist der Brechungsindex des Kerns größer als derjenige der Mantelschicht.

In einer Ausführungsform kann auch vorgesehen sein, dass das Funktionsschichtsystem unmittelbar auf dem optisch leitenden Kern befindlich ist. In dieser Ausführungsform umfasst der Lichtwellenleiter demnach keine äußere Mantelschicht. Mit anderen Worten kann z.B. eine reine Quarzglasfaser vorgesehen sein, wobei darauf direkt die Funktionsschichten aufgebracht sind und insbesondere durch eine letzte polymere Schicht abgeschlossen sein kann.

Die den Kern umgebende organische Mantelschicht, welche auch als Schlichte ausgebildet sein kann, umfasst vorzugsweise Polyamid (PA), Polyimid (PI) oder Polymethylmethacrylat (PMMA) oder Wachs, wachsähnliche Bestandteile oder Alkylsilan oder ist aus zumindest einem dieser Materialien gefertigt.

Wie ausgeführt kann ein auf der Mantelschicht befindliches Funktionsschichtsystem einen unterhalb der Leitschicht befindlichen Grundschichtbereich umfassen. Der Grundschichtbereich kann ausgebildet sein als eine auf der äußeren Mantelschicht aufgebrachte Schicht mit einem Oxid, insbesondere $SiO_2$, $TiO_2$, $Al_2O_3$, $SnO_2$, $HfO_2$ oder einem Borid, Carbid, Nitrid, Oxinitrid, Carbonitrid oder einem Metall, insbesondere Si, Ti, Mo oder Cr, oder als eine auf der äußeren Mantelschicht aufgebrachte Abfolge solcher Schichten. Mit anderen Worten kann die äußere Mantelschicht mit einer einzelnen oder mit mehreren Schichten, welche einen Grundschichtbereich bilden, beschichtet sein. Eine solche Schicht oder Schichtfolge kann etwa herstellbar oder hergestellt sein durch ein Beschichtungsverfahren, wie weiter unten näher ausgeführt ist. Ein Grundschichtbereich kann insbesondere ausgebildet sein als eine anorganische Haftvermittlerschicht auf Basis von Oxiden, wie $SiO_2$, $TiO_2$, $Al_2O_3$, $SnO_2$, $HfO_2$, usw., Boriden, Carbiden, Nitriden, Oxinitriden, Carbonitriden oder Metallen, wie z.B. Si, Ti, Mo oder Cr, als Einzelschicht oder als Schichtfolge aus diesen Stoffen. Bevorzugte Schichtsysteme umfassen $TiO_2$. Mit anderen Worten enthält ein Grundschichtbereich bevorzugt $TiO_2$ und umfasst insbesondere mehrere Schichten, wobei zumindest eine dieser Schichten $TiO_2$ enthält. Diese Beschichtungen können u.a. mittels einem Sputterprozess von einem sogenannten Sputtertarget appliziert werden, wobei diese Materialien als metallische Targets oder als teilkeramische Targets vorliegen können. Die Reinheit der Targets wird typischerweise mit 99% oder mehr angegeben. Geringere Reinheiten sind aber auch möglich. Hierbei sind dann ggf. höhere Schichtdicken erforderlich.

Gemäß einer anderen Ausführungsform kann ein Grundschichtbereich eine oberflächliche Schicht der äußeren Mantelschicht mit zumindest einer veränderten Oberflächeneigenschaft, insbesondere einer erhöhten Oberflächenenergie und/oder einer erhöhten Anzahl von Sauerstoffradikalen umfassen. Eine solche oberflächliche Teilschicht der Mantelschicht kann beispielsweise herstellbar oder hergestellt sein durch chemische oder physikalische Prozesse zur Veränderung der Oberflächeneigenschaften der Mantelschicht, insbesondere Plasmabehandlung (z.B. Niederdruck-Plasma oder atmosphärisches Plasma), UV-Behandlung, Lichtbogenentladung (Corona) und/oder durch chemische Behandlung, z.B. mittels alkalischen Reinigern im Ultraschallbad, oder eine Kombination solcher Verfahren, wie weiter unten näher beschrieben. Mit anderen Worten kann ein Grundschichtbereich oder eine unterste Schicht des Grundschichtbereichs durch einen die äußere Oberfläche umfassenden radialen Teil der äußeren Mantelschicht gebildet sein. Ein Grundschichtbereich kann also z.B. aus einer einzelnen Schicht bestehen, wobei diese einzelne Schicht mittels chemischer oder physikalischer Prozesse auf Basis der äußeren Mantelschicht des Lichtwellenleiters hinsichtlich der Oberflächeneigenschaften gebildet ist. Ein Grundschichtbereich kann aber auch aus einer Abfolge von Schichten bestehen, wobei z.B. die unterste Schicht eines Grundschichtbereichs durch chemische oder physikalische Prozesse auf Basis der äußeren Mantelschicht des Lichtwellenleiters hinsichtlich der Oberflächeneigenschaften gebildet ist und auf dieser untersten Schicht weitere Schichten aufgebracht sind.

Es kann demnach auch vorgesehen sein, dass ein Grundschichtbereich aus mehreren Schichten besteht, wobei eine unterste Schicht als eine solche oberflächliche Schicht der äußeren Mantelschicht ausgebildet ist und zumindest eine weitere darüber befindliche Schicht des Grundschichtbereichs als eine auf der äußeren Mantelschicht aufgebrachte Schicht ausgebildet ist. Ein solcher mehrschichtige Grundschichtbereich kann z.B. herstellbar oder hergestellt sein indem zuerst eine zumindest abschnittsweise Behandlung der Mantelschicht beispielsweise mittels Plasmabehandlung, UV Behandlung, Lichtbogenentladung und/oder chemischer Behandlung durchgeführt wird und danach eine Beschichtung der Mantelschicht mit einem Beschichtungsverfahren erfolgt. Durch die Behandlung wird insbesondere zum einen die Oberflächenenergie erhöht, zum anderen werden vorzugsweise freie Sauerstoffradikale erzeugt, welche eine gute Anhaftung folgender Beschichtungen gewährleisten. Die Behandlungen erfolgen bevorzugt vollflächig an der Faseroberfläche, d.h. der Oberfläche des Lichtwellenleiters mit Mantelschicht. Auf der durch Beschichtungsverfahren aufgebrachten Schicht können weitere Schichten aufgebracht sein.

Ein Grundschichtbereich weist vorzugsweise eine Dicke zwischen 5nm und 3000nm auf, bevorzugter zwischen 5nm und 1000nm, besonders bevorzugt zwischen 10nm und 100nm. Ferner können die einzelnen Schichten eines Grundschichtbereichs jeweils eine Dicke zwischen 5nm und 1000nm, bevorzugt zwischen 10nm und 100nm aufweisen. Beispielsweise kann ein zumindest zweischichtiger Grundschichtbereich vorgesehen sein, wobei die untere Schicht als eine oberflächliche Schicht der äußeren Mantelschicht ausgebildet ist und darüber eine Schicht mit einer Dicke zwischen 5nm und 1000nm, bevorzugt zwischen 10nm und 100nm, aufgebracht ist. Die Schichtdicke kann abhängig vom thermischen Ausdehnungskoeffizienten des zu beschichtenden Lichtwellenleiters bzw. seiner äußeren Polymerschicht im Vergleich zum thermischen Ausdehnungskoeffizienten der Leitschicht bestimmt werden.

Ein Grundschichtbereich kann insbesondere als Haftvermittlerschicht ausgebildet sein. Demnach kann vorgesehen sein, dass zwischen dem Grundschichtbereich und der darauf aufgebrachten Leitschicht eine größere Haftung besteht als sie bestehen würde zwischen der äußeren Mantelschicht und einer auf dieser aufgebrachten identischen Leitschicht. Eine gute Haftung ergibt sich z.B., wenn ein sogenannter Tape-Test (Druckfarbenhaftung) in Anlehnung an ASTM F 2252/ Sun Chemical-Hartmann PV 01 erfüllt werden kann. Hierbei wird ein Klebestreifen auf die beschichteten Fasern aufgebracht und unter einem definierten Winkel gleichmäßig abgezogen. Befindet sich nach dem Abziehen keine Beschichtung an dem Klebestreifen und zeigen sich bei der Beschichtung keine Delaminationen, so gilt der Test als bestanden. Die Haftung kann auch mit einem Haftungstest nach DIN 58196-6 (1995-07) überprüft werden. Mit anderen Worten kann eine erhöhte Haftung bestehen zwischen der Leitschicht, insbesondere der untersten Schicht der Leitschicht, und der darunter befindlichen obersten Schicht eines Grundschichtbereichs, welche ausgebildet sein kann als eine oberflächliche Schicht der äußeren Mantelschicht mit erhöhter Oberflächenenergie und/oder erhöhter Anzahl von Sauerstoffradikalen oder als eine auf der Mantelschicht durch Beschichtung aufgebrachten Schicht. Es ist auch möglich, dass ein Grundschichtbereich aus einer Mehrzahl von Schichten besteht, wobei zumindest eine oder eine jede oberhalb der untersten Schicht aufgebrachte Schicht eine höhere Haftung auf der darunter befindlichen Schicht aufweist als sie auf der Schicht, welche sich unter der darunter befindlichen Schicht befindet, aufweisen würde.

Ferner kann ein Grundschichtbereich als Barriereschicht ausgebildet sein. Demnach kann vorgesehen sein, dass ein Grundschichtbereich, insbesondere zumindest eine der auf der äußeren Mantelschicht durch Beschichtung aufgebrachten Schichten des Grundschichtbereichs ein Eindiffundieren von Polymerbestandteilen beispielsweise Säuren und/oder Sauerstoff und insbesondere von Ionen saurer oder alkalischer Lösungen in die Leitschicht hemmt oder blockiert. Der Grundschichtbereich kann demnach eine Permeation von z.B. Säuren, Sauerstoff oder auch anderen Bestandteilen der Luft in die untere Oberfläche der Leitschicht verzögern oder verhindern. Insbesondere bei der Aufbereitung medizinischer Produkte können alkalische Reiniger bzw. Desinfektoren zum Einsatz (z.B. NEODISHER® mit einem ph-Wert von etwa 11).

Ferner kann das Spülmittel Natriumhypochlorit (NaClO), ein Bleich- oder auch Desinfektionsmittel zum Einsatz kommen.

Ein Grundschichtbereich weist vorzugsweise einen thermischen Ausdehnungskoeffizienten auf, welcher zwischen dem thermischen Ausdehnungskoeffizienten der Mantelschicht und dem thermischen Ausdehnungskoeffizienten der Leitschicht liegt. Bei einem vorgegebenen thermischen Ausdehnungskoeffizienten (CTE) der Mantelschicht, welcher z.B. im Bereich von > 15*E-6 1/K liegen kann, können die Eigenschaften der Grundschichten abhängig von der verwendeten Leitschicht gewählt werden. Wird z.B. eine Molybdän Beschichtung als Leitschicht verwendet, welche typischerweise einen CTE von 5-6*E-6 1/K besitzt, kann ein Grundschichtbereich mit einem höheren CTE gewählt werden. Als Beispiel kann sich dann eine TiO2 Beschichtung mit einem CTE von 7-8*E-6 1/K anbieten. Vorzugsweise kann vorgesehen sein, dass der thermische Ausdehnungskoeffizient der Mantelschicht größer ist als der thermische Ausdehnungskoeffizient des Grundschichtbereichs. Ferner kann vorgesehen sein, dass der thermische Ausdehnungskoeffizient eines Grundschichtbereichs größer als oder gleich ist wie der thermische Ausdehnungskoeffizient der Leitschicht. Bei einem mehrschichtigen Grundschichtbereich gilt dies zumindest für eine ihrer Schichten. Außerdem kann vorgesehen sein, dass ein Grundschichtbereich eine Abfolge von Schichten umfasst, welche jeweils einen thermischen Ausdehnungskoeffizienten aufweisen, welche entsprechend der Abfolge der Schichten aufsteigen oder absteigen. Es kann auch durch quasi-kontinuierliche Veränderung der Zusammensetzung ein Gradient des thermischen Ausdehnungskoeffizienten (CTE) erzielt werden. Die Auswahl der Materialien eines Grundschichtbereichs kann abhängig vom thermischen Ausdehnungskoeffizienten des zu beschichtenden Lichtwellenleiters bzw. seiner äußeren Polymerschicht im Vergleich zum thermischen Ausdehnungskoeffizienten der Leitschicht bestimmt werden.

Eine auf dem Grundschichtbereich aufgebrachte Leitschicht umfasst vorzugsweise eine Schicht mit Titan, Silizium (ggf. mit n- oder p-leitenden Dotierstoffen dotiert zur Erhöhung der intrinsischen Leitfähigkeit), Aluminium, Gold, Silber, Molybdän, Wolfram oder Zirkon, insbesondere einer Legierung eines dieser Stoffe mit Ni, Zn, Y, Sn, Ge oder eine Abfolge solcher Schichten. Als besonders bevorzugt haben sich Ti und Mo gezeigt. Eine Schicht mit oder aus Molybdän ist demnach z.B. besonders bevorzugt. Mit anderen Worten kann die Leitschicht aus einer einzelnen Schicht oder aus einer Schichtfolge bestehen, wobei zumindest eine Schicht aus Titan, (dotiertem) Silizium, Aluminium, Gold, Silber, Molybdän, Wolfram, Zirkon oder aus Legierungen aus mindestens einer der genannten leitfähigen Stoffe, Ni und deren Legierungen, Zn, Y, Sn, Ge ist. Eine solche Schicht oder Schichtfolge kann beispielsweise herstellbar oder hergestellt sein durch ein Beschichtungsverfahren, wie weiter unten näher ausgeführt ist.

Eine auf dem Grundschichtbereich aufgebrachte Leitschicht kann ferner auch Ag, Cu, Cr, Ni, ITO umfassen oder daraus bestehen, wobei aber im medizintechnischen Bereich ggf. Anforderungen der Biokompatibilität und Zytotoxizität (ISO 10993-5:2009) zu beachten sind.

Die Leitschicht weist vorzugsweise eine Dicke zwischen 5nm und 6000nm auf, bevorzugter zwischen 5nm und 2000nm, besonders bevorzugt zwischen 10nm und 200nm. Ferner kann zumindest eine der Schichten oder eine jede der Schichten der Leitschicht eine Dicke zwischen 5nm und 2000nm, bevorzugt zwischen 10nm und 200nm aufweisen. Die Schichtdicke kann bestimmt sein in Abhängigkeit vom zu erzielenden elektrischen Widerstand bzw. Flächenwiderstand, wobei der Flächenwiderstand innerhalb bestimmter Schichtdickenbereiche sich umgekehrt proportional zur Schichtdicke der Leitschicht verhält. Eine Schichtdicke von zumindest 5nm hat den Vorteil, dass die Gefahr einer Inselbildung (nur bereichsweise Beschichtung) vermieden wird und daher eine geschlossene Schicht entsteht. Im Falle einer Inselbildung ergibt sich aufgrund der isolierenden Bereiche um die leitfähigen "Inseln" keine gleichmäßige Leitfähigkeit. Gegebenenfalls können jedoch auch Schichtdicken von zumindest 3nm ausreichend sein.

Die Leitschicht weist vorzugsweise einen Flächenwiderstand zwischen 0,01 und 1000, bevorzugt zwischen 0,01 und 100, besonders bevorzugt zwischen 0,01 und 50 Ohm/sqr auf (wobei die Einheit Ohm/sqr der Einheit Ohm entspricht). Unter dem Flächenwiderstand wird der spezifische elektrische Widerstand geteilt durch die Schichtdicke verstanden. Für den spezifischen elektrischen Widerstand gilt beispielsweise Ti: 8E-5 Ohm cm, Si: 2 Ohm cm, Au: 6E-8 Ohm cm, Ag: 1E-5 Ohm cm, Mo: 4,9 E-5 Ohm cm. Eine dünne 200nm Ti-Beschichtung zeigt einen Flächenwiderstand von 3,9 Ohm/sqr. Hieraus ergibt sich ein spezifischer Widerstand von 7,8*E-5 Ohm*cm.

Wie weiter oben bereits beschrieben kann ferner eine mittelbar oder unmittelbar auf der Leitschicht aufgebrachte Barriereschicht (Passivierungsschicht) vorgesehen sein, wobei diese Barriereschicht aus einer einzelnen Schicht oder einer Abfolge von Schichten bestehen kann. Die Barriereschicht ist vorzugsweise ausgebildet, ein Eindiffundieren von Sauerstoff und/oder Säuren/Basen, insbesondere von Ionen saurer oder alkalischer Lösungen, in die Leitschicht zu hemmen oder zu blockieren. Insbesondere bei der Aufbereitung medizinischer Produkte kommen mitunter alkalische Reiniger zum Einsatz (z.B. NEODISHER® mit einem ph-Wert von etwa 11).

Auch kann das Spülmittel Natriumhypochlorit (NaClO), ein Bleich- oder auch Desinfektionsmittel, zum Einsatz kommen. Die Barriereschicht kann demnach eine Permeation von z.B. Säuren, Sauerstoff oder auch anderen Bestandteilen der Luft in die obere Oberfläche der Leitschicht verzögern oder verhindern. Darüber hinaus kann die Barrierebeschichtung dafür sorgen, dass mechanische Angriffe auf die Leitschicht oder auch die Faser reduziert werden können und bildet damit einen mechanischen Schutzfilm. In Kombination mit einem entsprechenden Grundschichtbereich kann die Leitschicht somit beidseits vor unerwünschter Diffusion geschützt sein.

Vorzugsweise weist die Barriereschicht oder zumindest eine der Schichten der Barriereschicht eine Härte von mindestens 800 HV, vorzugsweise mindestens 1200 HV, besonders bevorzugt mindestens 2000 HV gemäß der Prüfnorm DIN EN ISO 14577-4:2007-8 auf. Die Barriereschicht kann somit auch als eine mechanische Schutzschicht für die Leitschicht, insbesondere für eine metallische Schicht, und/oder als Schutz für den Lichtwellenleiter bzw. die Mantelschicht (Buffermaterial) ausgebildet sein. Insbesondere schützen Hartstoffmaterialien aus z.B. Carbiden oder Nitriden aufgrund deren erhöhten Härte, wie z.B. AIN: HV bis ca. 2000, Si3N4: HV bis ca. 2500.

Die Barrierschicht umfasst vorzugsweise eine auf der Leitschicht aufgebrachte Schicht mit einem Nitrid, insbesondere $Si_3N_4$, BN, AIN, TiN, AISiN, SiON, SiAlON oder einer Legierung eines dieser Stoffe, oder mit einem Oxid, insbesondere Oxide von Si, Al, Ti, Zr, Zn, Sn, Ta, Nb, Y oder einem ternären System aus zumindest einem dieser Stoffe, oder mit einem Carbid, Borid, Oxinitrid, Carbonitrid, oder eine auf der Leitschicht aufgebrachte Abfolge solcher Schichten. Als besonders geeignet zeigen sich Schichten mit $TiO_2$, TiN, $Si_3N_4$ oder $SiO_2$. Aufgrund der oben genannten CTE-Zusammenhänge sind Barrierebeschichtungen mit vergleichbarem CTE zur Leitschicht bevorzugt zu verwenden. Mit anderen Worten kann der thermische Ausdehnungskoeffizient der Barriereschicht dem 0,5-fachen bis 2-fachen, bevorzugt 0,75-fachen bis 1,25-fachen des thermischen Ausdehnungskoeffizienten der Leitschicht entsprechen. Ferner kann auch die Barriereschicht aus einer einzelnen Schicht oder aus einer Schichtfolge bestehen. Vorzugsweise ist zumindest eine Schicht der Barriereschicht aus Nitrid, z.B. $Si_3N_4$, BN, AIN, TiN, AISiN oder einer Legierung aus einem genannten Nitriden. Eine solche Schicht eignet sich insbesondere zur Hemmung der Diffusion von Luftsauerstoff und hat außerdem den Vorteil, dass sie verhältnismäßig hart ist. Ferner vorzugsweise ist zumindest eine Schicht, insbesondere eine weitere Schicht, aus Oxid, insbesondere Oxide von Si, Al, Ti, Zr, Zn, Sn, Ta, Nb, oder einem ternären System aus zumindest einem dieser Stoffe. Eine solche Schicht bildet insbesondere eine gute Barriere gegenüber Säuren. Für eine der Schichten, vorzugsweise eine weitere Schicht, können Carbide, Boride, Oxinitride, Carbonitride zum Einsatz kommen. Darüber hinaus können für die Barriereschicht und/oder eine der Schichten der Barriereschicht grundsätzlich auch die Materialien zum Einsatz kommen, welche auch für den Grundschichtbereich genutzt werden können, wobei diese sich insbesondere nach den chemischen bzw. atmosphärischen Anforderungen an eine derartige optisch-elektrische Leiteranordnung/Faser bzw. an Bauteile mit einer derartigen optisch-elektrische Leiteranordnung/Faser richten. Eine Schicht oder Schichtfolge kann beispielsweise herstellbar oder hergestellt sein durch ein Beschichtungsverfahren, wie weiter unten näher ausgeführt ist.

Eine Barriereschicht, insbesondere wie vorstehend beschrieben, kann demnach eine auf der Leitschicht als Beschichtung aufgebrachte Schicht umfassen. Es kann aber alternativ oder zusätzlich auch vorgesehen sein, dass eine Barriereschicht, insbesondere wie vorstehend beschrieben, eine oberflächliche Schicht der Leitschicht mit zumindest einer veränderten Materialeigenschaft umfasst oder als solche ausgebildet ist. Beispielsweise kann eine Barriereschicht auch als, insbesondere natürliche, Oxidationsschicht der Leitschicht (z.B. bei einer Aluminium-Leitschicht), und/oder als Kontaminationsfilm (Feuchtebelag, Partikel-Kontamination und/oder Schmutz) auf der Leitschicht ausgebildet sein.

Ein Grundschichtbereich, die Leitschicht und/oder eine ggf. vorhandene Barriereschicht erstrecken sich zumindest teil- oder abschnittsweise in axialer Richtung des Lichtwellenleiters.

Ein Grundschichtbereich kann eine amorphe Struktur aufweisen, insbesondere wenn der Grundschichtbereich als eine oberflächliche Teilschicht der Mantelschicht ausgebildet ist. Ein Grundschichtbereich kann auch eine kristalline oder polykristalline Struktur aufweisen, insbesondere, wenn der Grundschichtbereich eine durch Beschichtung aufgebrachte Schicht umfasst. Die Leitschicht und/oder eine ggf. vorhandene Barriereschicht kann ebenfalls eine kristalline oder polykristalline oder ggf. auch amorphe Struktur aufweisen. Bevorzugt sind für Grundschichtbereich und Barriereschicht amorphe Schichten, um eine besonders gute Diffusionsbarriere zur gewährleisten. Als typische Beispiele für amorphe Diffusionsbarrieren sind $SiO_2$, $Si_3N_4$, $Al_2O_3$, $AlSiO_x$ oder $BN$ zu nennen, als typische Beispiele für kristalline Barrierebeschichtungen sind anatases oder rutiles $TiO_2$, $\gamma$-$Al_2O_3$ oder kristallines $AlN$ zu nennen. Insbesondere sind aber auch Mischphasen aus amorph und kristallin zu nennen.

Die optisch-elektrische Leiteranordnung kann zumindest teil oder abschnittsweise noch eine Einschlauchung aufweisen, also von einem letzten Außenmantel umgeben sein, wobei dieser letzte Außenmantel fest oder lose umschließend ausgebildet sein kann. Es kann vorgesehen sein, dass ein distaler Abschnitt der optisch-elektrischen Leiteranordnung abisoliert wird oder verbleibt. Die Einschlauchung kann sich demnach überwiegend entlang der optisch-elektrischen Leiteranordnung erstrecken, wobei jedoch ein Teil der optisch-elektrischen Leiteranordnung, insbesondere ein Ende der Anordnung, keine Einschlauchung aufweist.

Die Erfindung betrifft ferner eine Vorrichtung zur Erfassung des Eintauchens einer optisch-elektrischen Leiteranordnung in ein leitfähiges Medium, insbesondere innerhalb eines tierischen oder menschlichen Körpers, wobei die Vorrichtung ein optisch-elektrisches Leitersystem sowie eine Auswerteeinheit umfasst.

Das optisch-elektrische Leitersystem ist gemäß den vorstehenden Ausführungen ausgebildet. Es umfasst zumindest: eine optisch-elektrische Leiteranordnung umfassend einen Lichtwellenleiter mit einer äußeren, organischen Mantelschicht, und eine unmittelbar oder mittelbar auf der äußeren Mantelschicht des Lichtwellenleiters aufgebrachte Leitschicht, und ferner: eine zumindest bereichsweise elektrisch leitfähige Adapterhülse, welche die optisch-elektrische Leiteranordnung mechanisch erfasst und deren Leitschicht elektrisch kontaktiert, derart, dass die optisch-elektrische Leiteranordnung in das leitfähige Medium eintauchbar ist. Die Auswerteeinheit ist mit der zumindest bereichsweise elektrisch leitfähigen Adapterhülse einerseits und einem, insbesondere distalen, Ende der optisch-elektrische Leiteranordnung und/oder dem leitfähigen Medium elektrisch verbindbar oder verbunden, und dazu eingerichtet, eine Impedanz oder Impedanzänderung zwischen der Adapterhülse und dem Ende der optisch-elektrische Leiteranordnung und/oder dem Medium zu bestimmen, wenn die optisch-elektrische Leiteranordnung in das leitfähige Medium eingetaucht ist oder wird. Auf diese Weise kann ein Eintauchen der optisch-elektrischen Leiteranordnung in das Medium erfasst werden und/oder eine Eintauchtiefe der optisch-elektrischen Leiteranordnung in das Medium bestimmt werden. Weiterhin kann die Vorrichtung zur Erfassung des Eintauchens derart ausgebildet sein, dass, wenn ein Eintauchen der optisch-elektrischen Leiteranordnung in das Medium erfasst wird und/oder dass in Abhängigkeit von der Eintauchtiefe, das Einkoppeln von Licht in den Lichtwellenleiter gesteuert wird. Beispielsweise kann das Einkoppeln von Licht aktiviert werden, sobald die optisch-elektrische Leiteranordnung in das Medium eingetaucht ist oder ausreichend weit eingetaucht ist. Hierzu kann die Vorrichtung ggf. ein Gerät oder eine Handstück mit einer Anschlussfassung zum Einsetzen der Adapterhülse sowie einer Lichtquelle zum Einkoppeln von Licht in den Lichtwellenleiter umfassen.

Die Erfindung betrifft ferner ein Verfahren zur Herstellung eines optisch-elektrischen Leitersystems wie vorstehend beschrieben.

Dabei wird eine zumindest bereichsweise elektrisch leitfähigen Adapterhülse bereitgestellt oder hergestellt. Ferner wird eine optisch-elektrischen Leiteranordnung bereitgestellt oder hergestellt, derart, dass die optisch-elektrischen Leiteranordnung zumindest umfasst: einen Lichtwellenleiter mit einer äußeren, organischen Mantelschicht, und eine unmittelbar oder mittelbar auf der äußeren Mantelschicht des Lichtwellenleiters aufgebrachte Leitschicht bestehend aus einer einzelnen Schicht oder einer Abfolge von Schichten. Die optisch-elektrischen Leiteranordnung wird dann in die Adapterhülse eingeführt, derart, dass die Adapterhülse die optisch-elektrische Leiteranordnung mechanisch erfasst und deren Leitschicht elektrisch kontaktiert.

Vorzugsweise umfasst die bereitgestellte oder hergestellte Adapterhülse einen Fixierungsabschnitt, um die optisch-elektrische Leiteranordnung mechanisch zu erfassen und es wird in den Fixierungsabschnitt ein Kleber eingebracht, um die optisch-elektrische Leiteranordnung zusätzlich zu fixieren.

Erfindungsgemäß umfasst die bereitgestellte oder hergestellte Adapterhülse einen elektrisch leitfähigen Kontaktierungsabschnitt, um die Leitschicht der optisch-elektrischen Leiteranordnung elektrisch zu kontaktieren und es wird in den Kontaktierungsabschnitt ein elektrischer Leitkleber eingebracht, um die Leitschicht der optisch-elektrischen Leiteranordnung elektrisch zu kontaktieren.

Nachfolgend soll noch näher auf die Herstellung der optisch-elektrischen Leiteranordnung selbst eingegangen werden.

Es kann eine Herstellung einer optisch-elektrischen Leiteranordnung vorgesehen sein, wobei ein Lichtwellenleiter mit einer äußeren Mantelschicht bereitgestellt wird und die Mantelschicht des Lichtwellenleiters mit einem Funktionsschichtsystem beschichtet wird. Das Beschichten mit dem Funktionsschichtsystem umfasst zunächst das Erzeugen eines Grundschichtbereichs bestehend aus einer einzelnen Schicht oder einer Abfolge von Schichten und danach das Aufbringen (auf dem Grundschichtbereich) einer elektrisch leitenden Leitschicht bestehend aus einer einzelnen Schicht oder einer Abfolge von Schichten.

Das Erzeugen des Grundschichtbereichs kann zum einen das Vorbehandeln der Mantelschicht des Lichtwellenleiters umfassen, um eine oberflächliche Schicht mit zumindest einer veränderten Oberflächeneigenschaft, insbesondere einer erhöhten Oberflächenenergie und/oder einer erhöhten Anzahl von Sauerstoffradikalen zu erzeugen und/oder Rückstände zu entfernen. Das Erzeugen des Grundschichtbereichs kann zum anderen das Aufbringen (auf der äußeren Mantelschicht) einer Schicht oder eine Abfolge von Schichten umfassen, wobei insbesondere Kathoden-Zerstäubung, Hochfrequenz-Zerstäubung, Reaktiv-Zerstäubung und/oder Magnetron-Zerstäubung zum Einsatz kommt. Neben Kathoden-Zerstäubung (Sputtern) können auch andere Beschichtungsverfahren, insbesondere Vakuumverfahren (z.B. Aufdampfen, chemische Gasphasenabscheidung (CVD, z.B. PECVD, insb. PICVD)) zum Einsatz kommen. Als weitere Beschichtungsverfahren zum Aufbringen einer oder mehrerer Schichten des Grundschichtbereichs sind darüber hinaus Verfahren aus der flüssigen Phase, wie z.B. Tauchbeschichtung oder Sprühbeschichtungen möglich. Hierbei können weitere Funktionen wie Reibwertreduzierung mit funktionalisiert werden.

Das Vorbehandeln der Mantelschicht, um den Grundschichtbereich oder z.B. die unterste Schicht des Grundschichtbereichs zu bilden, kann mittels chemischer oder physikalischer Prozesse zur Veränderung der Oberflächeneigenschaften der Mantelschicht erfolgen. Ein Vorbehandeln der Mantelschicht, d.h. der zu beschichtenden Oberfläche, ermöglicht eine gute Schichthaftung. Insbesondere können mit einer Plasmavorbehandlung (Niederdruck-Plasma oder atmosphärisches Plasma), einer Lichtbogenentladung (Corona) und/oder auch einer chemischen Vorbehandlung, z.B. mittels alkalischen Reinigern im Ultraschallbad, zumindest temporär hohe Oberflächenenergien erzeugt werden, so dass dadurch die Haftung deutlich verbessert werden kann. Mittels Plasmen lassen außerdem Fette, Öle oder ähnlich Rückstände entfernen und zusätzlich Sauerstoffradikale aktivieren. Es können aber auch Vorbehandlungsmethoden kombiniert werden.

Es kann auch vorgesehen sein, dass zuerst eine untere Schicht des Grundschichtbereichs gebildet wird, indem die Mantelschicht des Lichtwellenleiters wie vorstehend beschrieben vorbehandelt wird, und danach auf diese untere Schicht zumindest eine weitere Schicht des Grundschichtbereichs aufgebracht wird, wobei insbesondere die genannten Zerstäubungsmethoden (oder andere Beschichtungsverfahren) zum Einsatz kommen. Insbesondere kann mit dem Verfahrensschritt ein Grundschichtbereich erzeugt werden, wie sie im Zusammenhang mit der optisch-elektrischen Leiteranordnung beschrieben ist.

Das Aufbringen der elektrisch leitenden Leitschicht auf dem Grundschichtbereich erfolgt ebenfalls vorzugsweise mittels Kathoden-Zerstäubung, Hochfrequenz-Zerstäubung, Reaktiv-Zerstäubung und/oder Magnetron-Zerstäubung. Neben Kathoden-Zerstäubung (Sputtern) können wiederum auch andere Beschichtungsverfahren, insbesondere Vakuumverfahren (z.B. Aufdampfen, chemische Gasphasenabscheidung (CVD, z.B. PECVD, insb. PICVD)) zum Einsatz kommen. Mit dem Verfahrensschritt kann insbesondere eine Leitschicht auf den Grundschichtbereich aufgebracht werden, wie sie im Zusammenhang mit der optisch-elektrischen Leiteranordnung beschrieben ist.

Das Beschichten der Mantelschicht mit einem Funktionsschichtsystem kann ferner umfassen, dass auf die Leitschicht eine Barriereschicht (Passivierungsschicht) aufgebracht wird, wobei die Barriereschicht als einzelne Schicht oder als Abfolge von Schichten aufgebracht werden kann und hierzu jeweils insbesondere wiederum Kathoden-Zerstäubung, Hochfrequenz-Zerstäubung, Reaktiv-Zerstäubung und/oder Magnetron-Zerstäubung (oder andere Beschichtungsverfahren, wie z.B. die genannten Vakuumverfahren oder die genannten Verfahren aus der flüssigen Phase, wie z.B. Tauchbeschichtung oder Sprühbeschichtungen) zum Einsatz kommen kann. Die Barriereschicht kann auch zur Reibwertreduzierung dienen. Mit dem Verfahrensschritt kann insbesondere eine Barriereschicht auf der Leitschicht aufgebracht werden, wie sie im Zusammenhang mit der optisch-elektrischen Leiteranordnung beschrieben ist.

Die genannten Beschichtungsverfahren, insbesondere Kathoden-Zerstäubungs-Verfahren, mit denen Schichten des Grundschichtbereichs, der Leitschicht und/oder der Barriereschicht aufgetragen werden können, erfolgt vorzugsweise bei Temperaturen von unter 50°C. Diese relativ niedrige Temperatur hat insbesondere den Vorteil, dass Lichtwellenleiter mit äußerer Polymerschicht beschichtet werden können.

Bevorzugt erfolgt das Erzeugen und/oder Aufbringen zumindest zweier Schichten im Vakuum und ohne Vakuumbruch. Es kann demnach beispielsweise im Vakuum zuerst der Grundschichtbereich erzeugt werden und danach in demselben Vakuum die Leitschicht aufgebracht werden. Ferner kann danach in demselben Vakuum noch eine Barriereschicht aufgebracht werden.

Ferner kann eine medizintechnische Vorrichtung vorgesehen sein, insbesondere ein Endoskop, insbesondere zur Zahnbehandlung, umfassend ein optisch-elektrisches Leitersystem wie weiter oben beschrieben. Eine solche medizintechnische Vorrichtung kann z.B. ausgebildet sein als ein medizintechnisches Faserbauteil mit einem optisch-elektrischen Leitersystem umfassend einen Lichtwellenleiter, etwa einer Quarzfaser, wobei der Lichtwellenleiter mit einer Leitschicht bzw. einem Funktionsschichtsystem beschichtet ist. Eine solche Vorrichtung kann beispielsweise ausgebildet sein für endoskopische Anwendungen oder Anwendungen im Dentalbereich (Zahnbehandlung).

Ferner kann eine industrietechnische Vorrichtung vorgesehen sein, insbesondere eine Einrichtung zur Füllstandsüberwachung, insbesondere für Behälter oder Bioreaktoren, umfassend ein optisch-elektrische Leitersystem wie vorstehend beschrieben. Eine solche industrietechnische Vorrichtung kann beispielsweise für industrielle Anwendungen, z.B. für Füllstandssensoren in Behältern, Bioreaktoren oder Sicherheitsüberwachungen vorgesehen sein. Es wird insbesondere eine Füllstandüberwachung ermöglicht, bei der eine Impedanz-Änderung, je nachdem, ob die Faser in ein Medium eintaucht oder nicht, detektierbar und auswertbar ist. Außerdem wird eine Eindringtiefenmessung ermöglicht, bei der eine Impedanz-Änderung, je nachdem wie weit die Faser in ein umgebendes Medium eintaucht, detektierbar und auswertbar ist.

Schließlich kann eine Verwendung eines optisch-elektrischen Leitersystem für industrietechnische Anwendungen, insbesondere Anwendungen zur Füllstandsüberwachung oder Sicherheitsüberwachungen vorgesehen sein, oder auch eine Verwendung eines optisch-elektrischen Leitersystems zur Messung einer Eindringtiefe der optisch-elektrischen Leiteranordnung in ein Medium durch Messung einer Impedanz der Leitschicht im umgebenden Medium.

Nachfolgend werden Ausführungsbeispiele der Erfindung anhand der Figuren beschrieben. Dabei zeigen:

Fig. 1:    einen Querschnitt durch eine optisch-elektrische Leiteranordnung (1) mit einem optisch leitenden Kern (11), einer Umhüllung (12), einer Mantelschicht (13) und einem Funktionsschichtsystem (20),

Fig. 2:    einen Querschnitt durch eine optisch-elektrische Leiteranordnung (1) mit einem optisch leitenden Kern (11), einer Mantelschicht (13) und einem Funktionsschichtsystem (20),

Fig. 3:    einen Querschnitt durch eine Vorrichtung zur Eintaucherfassung (200) umfassend ein optisch-elektrischen Leitersystem (100) mit einer optisch-elektrische Leiteranordnung (1) und einer Adapterhülse (30), welche die optisch-elektrische Leiteranordnung (1) mechanisch erfasst und deren Leitschicht (22) elektrisch kontaktiert,

Fig. 4a:    Ersatzschaltbild für die Gesamtimpedanz eines optisch-elektrisches Leitersystem (100),

Fig. 4b:    Vereinfachtes Ersatzschaltbild für die Gesamtimpedanz eines optisch-elektrisches Leitersystem (100),

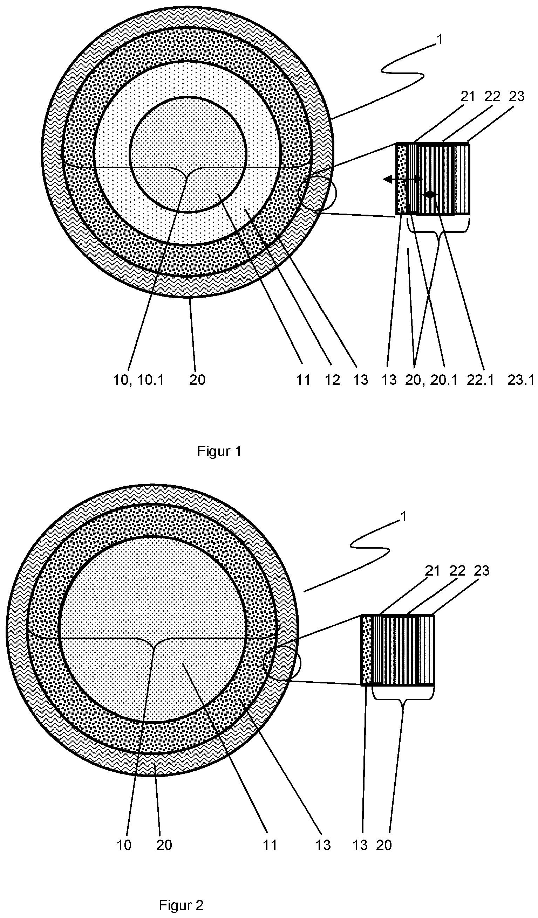

Figur 1 zeigt eine optisch-elektrische Leiteranordnung 1 mit einem Lichtwellenleiter 10 mit seinem Durchmesser 10.1. Der Lichtwellenleiter 10 weist einen Kern 11 (Core) aus Quarzglas auf und eine Umhüllung 12 (Cladding), hier ebenfalls aus Quarzglas. Der Brechungsindex n1 des Kerns 11 ist größer ist als der Brechungsindex n2 der Umhüllung 12. Zusätzlich und insbesondere als mechanischer Schutz ist eine als Polymerschicht/Polymermantel ausgebildete Mantelschicht 13 (Buffer) vorgesehen. Die Mantelschicht 13 umfasst, wie für derartige Lichtwellenleiter üblich, Polyimid, PMMA oder Polyamid, oder besteht aus zumindest einem dieser Materialien.

Die optisch-elektrische Leiteranordnung 1 weist auf der Mantelschicht 13 ein Funktionsschichtsystem 20 auf, welches in diesem Beispiel aus einem als Haftvermittlerschicht ausgebildeten Grundschichtbereich 21 direkt auf der Mantelschicht 13, einer Leitschicht 22 und einer äußeren Barriereschicht (Passivierungsschicht) 23 besteht. Das Funktionsschichtsystem 20 auf der äußeren Mantelschicht 13 ist mittels Kathoden-Zerstäubung (Sputtern) oder einem anderen Vakuumverfahren (z.B. Aufdampfen) herstellbar. Das Funktionsschichtsystem 20 weist dabei eine Schichtdicke 20.1 auf.

Figur 2 zeigt eine optisch-elektrische Leiteranordnung 1 mit einem Lichtwellenleiter 10, wobei in diesem Fall der Lichtwellenleiter 10 einen Kern 11 und eine den Kern 11 unmittelbar umgebende Mantelschicht 13 aufweist. Der Brechungsindex n1 des Kerns 11 ist in diesem Fall geringfügig größer ist als der Brechungsindex n2 der Mantelschicht 13. Die Mantelschicht 13 ermöglicht somit eine Totalreflexion an der Grenzschicht zum Kern 11 und damit eine Lichtleitung. Zugleich kann die Mantelschicht 13 als mechanische Schutzschicht dienen.

Die optisch-elektrische Leiteranordnung 1 weist darüber hinaus ein Funktionsschichtsystem 20 auf, welches in gleicher Weise ausgebildet und herstellbar sein kann wie bei der Figur 1.

Wie bereits ausgeführt kann das Funktionsschichtsystem 20 mehrere Einzelschichten umfassen. Diese sind hier ein als Haftvermittlerschicht ausgebildeter Grundschichtbereich 21, die eigentliche Leitschicht 22 und eine optionale Barriereschicht (Passivierungsschicht) 23. Hinsichtlich der Herstellung einer solchen optisch-elektrischen Leiteranordnung kann die gesamte Schichtfolge in einem Batch-Zyklus ohne Unterbrechung des Vakuum-Prozesses hintereinander durchgeführt werden, so dass auch kostengünstig mehrere Bauteile mit derartigen optisch-elektrischen Leiteranordnungen 1 parallel beschichtet werden können.

Nachfolgend werden weitere Ausführungsbeispiele der Herstellung einer optisch-elektrischen Leiteranordnung beschrieben.

Beispiel 1: Optisch-elektrische Leiteranordnung 1 mit einem als Quarzfaser ausgebildeten Lichtwellenleiter 10 mit einem Kern 11 dessen Durchmesser 150μm beträgt, einer Umhüllung 11, deren Durchmesser 180μm beträgt und eine als Polymerschicht ausgebildete Mantelschicht 13 aus Polyimid mit einem äußeren Durchmesser des Lichtwellenleiters 10 von insgesamt ca. 210 μm.

Es wurde ein Grundschichtbereich erzeugt, indem die Mantelschicht einer Vorbehandlung unterzogen wurde. Hierbei kam eine Ultraschallreinigung mit einem alkalischen, einem Neutralreiniger und einer IR-Trocknung zum Einsatz.

Es wurde eine Leitschicht aufgebracht bestehend aus einer Titanbeschichtung der Dicke 15nm, welche mittels DC-Magnetronsputtering hergestellt wurde. Die Beschichtung erfolgte im Vakuum bei einem Prozessdruck von unter 1E-2 mbar. Das Sputtertarget wurde mit einer Reinheit von 99% gewählt. Der Mindestabstand zwischen Substrat und Target wurde mit 5cm gewählt, wobei der Lichtwellenleiter in das Plasma hinein ragt.

Mittels einem 4-Punkt-Messgerät zur Bestimmung des Flächenwiderstands wurde ein Wert von 10 Ohm/sqr (wobei die Einheit Ohm/sqr der Einheit Ohm entspricht) gemessen. Dies entspricht einen spezifischen Widerstand von 1,5 E-5 Ohm cm. Die Haftung des Schichtsystems wurde mit einem Haftungstest nach DIN 58196-6 (1995-07) überprüft. Hierbei ergaben sich keine Ablösungen des Funktionsschichtsystems von dem Lichtwellenleiter.

Beispiel 2: Ein als Quarzfaser ausgebildeter Lichtwellenleiter mit einer Mantelschicht aus Polyimid wurde mittels einem Atmosphärenplasma in Form einer Corona Entladung gereinigt und voraktiviert. Anschließend erfolgte eine Beschichtung mittels reaktivem Mittelfrequenzplasma einer Siliziumoxid-Beschichtung, welche ohne Vakuumbruch mit einer leitfähigen Molybdänbeschichtung der Schichtdicke 24nm versehen wurde. Anschließend erfolgte eine Passivierung dieser Schicht ohne Vakuumbruch mittels reaktivem Magnetronsputtern mit einer Siliziumnitrid-Beschichtung der Schichtdicke 100nm.

In einer anschließenden Prüfung des Flächenwiderstands wurde ein Flächenwiderstand von 5 Ohm/sqr mittels einem induktiven Messverfahren, einem Wirbelstrommessgerät, bestimmt. Gemäß dem oben erwähnten Haftungstest konnten keine Delaminationen festgestellt werden.

Beispiel 3: Ein als Quarzfaser ausgebildeter Lichtwellenleiter mit einer Mantelschicht aus Polyimid wird mittels Ultraschallreinigung gemäß Beispiel 1 gereinigt. Die direkt auf die Mantelschicht aufgebrachte Leitschicht umfasst Molybdän mit einem Flächenwiderstand von 10 Ohm/sqr. Zum Schutz der Molybdänbeschichtung erfolgt optional die Beschichtung einer Barrierebeschichtung aus TiO2. Beide Beschichtungen werden in einem Magnetron Sputterprozess im Vakuum hergestellt, wobei die Lichtwellenleiter in das Plasma hineinragen und damit eine nahezu homogene Beschichtung erzeugt wird. Die Beschichtungen erfolgen im Fall der Molybdänbeschichtung von einem metallischen Sputtertarget der Reinheit 3N, im Falle der TiO2 Beschichtung von einem metallischen Target oder einem teilkeramischen Target unter der Zugabe von Sauerstoff. Die TiO2 Beschichtung ist in diesem Falle teilweise amorph und teilweise anatase ausgebildet. In einem anschließenden mechanischen Belastungstest, bei welchem Aluminium Prüfkörper der Masse 22,5g über die Länge der Lichtwellenleiter gezogen werden zeigen sich anhand Lichtmikroskopischer Aufnahmen unter bis zu 100facher Vergrößerung keine Kratzer oder Delaminationen der metallischen Beschichtung.

Beispiel 4: In einem weiteren Ausführungsbeispiel wird ein als Quarzfaser ausgebildeter Lichtwellenleiter mit einer Mantelschicht aus Polyimid mittels nasschemischer Reinigung vorbehandelt. Danach erfolgt das Aufbringen von Schichten sowohl des Grundschichtbereichs als auch der Leitschicht unter Zugabe von Sauerstoff und Argon. Um eine verbesserte Haftung zwischen Mantelschicht und leitfähiger Schicht zu gewährleisten wird dazwischen eine Haftvermittlerschicht aus TiO2 ausgebildet, wobei zu dessen Herstellung das Verhältnis von Sauerstoff zu dem Gesamtfluss aus Sauerstoff und Argon kleiner 0,4 beträgt. Anschließend wird als leitfähige Beschichtung eine metallische Titan-Beschichtung des Flächenwiderstandes von 1 Ohm/sqr beschichtet, wobei zu dessen Herstellung das Verhältnis von Sauerstoff zu dem Gesamtfluss aus Sauerstoff und Argon kleiner 0,1 beträgt. Als zusätzliche Passivierung wird eine weitere TiO2-Beschichtung appliziert, wobei zu dessen Herstellung das Verhältnis von Sauerstoff zu dem Gesamtfluss aus Sauerstoff und Argon kleiner 0,7 beträgt.

Das Verhältnis von Sauerstoff zum Gesamtfluss (siehe oben genannte beispielhafte Verhältnisse) gibt an, wie nah sich das Ergebnis an dem Metallcharakter oder dem dielektrischen Charakter der TiO2-Schicht befindet.

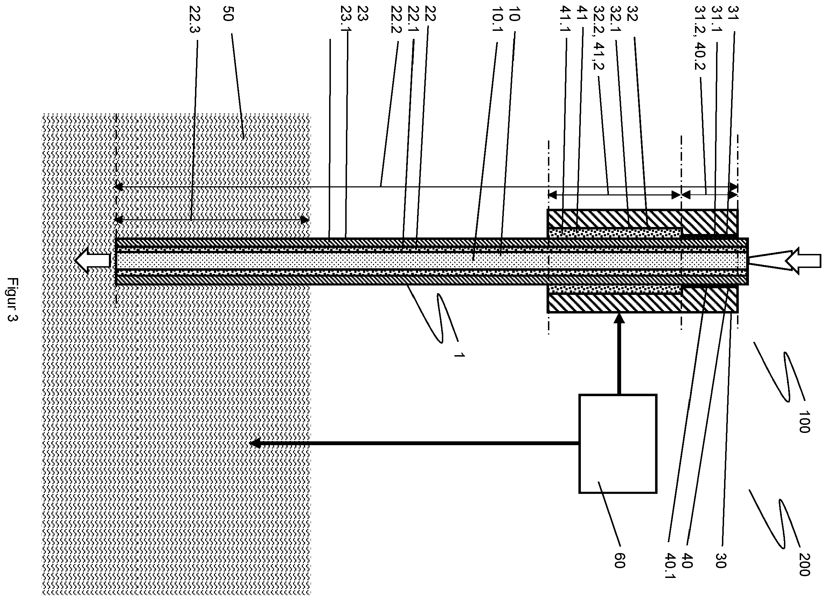

Fig. 3 zeigt eine Vorrichtung 200 zur Erfassung des Eintauchens einer optisch-elektrischen Leiteranordnung 1 eines optisch elektrischen Leitersystems 100 in ein leitfähiges Medium 50. Eine solche Vorrichtung 200 entspricht somit einer bevorzugten Anwendung einer solchen optisch-elektrischen Leiteranordnung 1 zur Füllstandsbestimmung bzw. zur Bestimmung einer Eindringtiefe 22.3 in eine leitfähige Flüssigkeit 50. Dazu ist der Lichtwellenleiter 10 mit seinem Funktionsschichtsystem 20 in einer elektrisch leitfähigen Adapterhülse 30 befestigt und bildet mit dieser gemeinsam ein optisch-elektrisches Leitersystem 100. Die Adapterhülse ist im gezeigten Ausführungsbeispiel mit einer elektrischen Auswerteeinheit 60 verbunden mit der Wechselspannungssignale zur Impedanzmessung mit einer bestimmten Frequenz f in die Hülse eingekoppelt werden können. Weiterhin ist die Auswerteeinheit 60 über die Erde oder direkt, wie in Figur 3 gezeigt, mit der Flüssigkeit 50 leitfähig verbunden.

Die elektrisch leitfähige Adapterhülse 30, welche beispielsweise aus Edelstahl oder Neusilber bestehen kann, weist einen Fixierungsabschnitt 31 mit seinem Durchmesser 31.1 und seiner Länge 31.2 zur Aufnahme der optisch-elektrischen Leiteranordnung 1 auf. Weiterhin weist die Adapterhülse 30 einen separaten Kontaktierungsabschnitt 32 mit seinem Durchmesser 32.1 und seiner Länge 32.2 auf. Die Durchmesser 31.1 und 32.1 sowie auch die Längen 31.2 und 32.2 können sich dabei unterscheiden. Bevorzugt weist der Fixierungsabschnitt 31 einen geringen Durchmesser 31.1 auf, welcher nur geringfügig größer ist als der Durchmesser des kompletten Lichtwellenleiters 10 mit seinem Funktionsschichtsystem 20, so dass bei einer Verklebung mit einem Kleber 40 eine möglichst eng tolerierte Lage und mit geringem Zentrizitätsfehler erzielt werden kann, damit eine optimale Lichteinkopplung in den optisch leitenden Kern 11 des Lichtwellenleiters 10 (vergl. Fig. 1 bzw. 2) erreicht werden kann. In der Regel wird hier auf den Kern 11 fokussiertes Laserlicht eingekoppelt. Oft werden dazu spezielle, sehr dünn flüssige Kleber 40 verwendet, die aufgrund der Kapillarwirkung den so erzeugten Kleberspalt ausfüllen können.

Hinsichtlich einer elektrischen Ankopplung ist es erforderlich, zusätzlich im Kontaktierungsabschnitt 32 einen Leitkleber 41 vorzusehen. Bei diesem Klebertyp handelt es sich i.d.R. um silbergefüllte Epoxid-Kleber, die allerdings einen größeren Kleberspalt benötigen. Daher ist der Durchmesser 32.1 des Kontaktierungsabschnitts 32 der Adapterhülse 30 größer als der Durchmesser 31.1 des Fixierungsabschnitts 31 der Adapterhülse 30.

Das optisch-elektrische Leitersystem, d.h. die optisch-elektrischen Leiteranordnung 1 mit der Adapterhülse 30 und den verwendeten Klebern 40, 41 bilden nun eine von der Frequenz f abhängige Impedanz aus, die sich bei Berührung mit der Flüssigkeit 50 bzw. beim Eintauchen in diese ändert. Dabei sind bei der Kontaktierung der optisch-elektrischen Leiteranordnung 1 in der Adapterhülse 30 auf geringstmögliche Übergangsimpedanzen, sowohl kapazitiv als auch infolge Ohm'scher Leitung, zu achten, um eine genügend sensitive Erfassung der Füllstandhöhe der Flüssigkeit bzw. der Eintauchtiefe 22.3 zu ermöglichen.

Betrachtet man diese Anordnung bei typ. Frequenzen im Bereich von einigen Hundert Hertz bis etwa 100 kHz, können induktive Impedanzen vernachlässigt werden, so dass sich ein elektrisches Ersatzschaltbild im Wesentlichen aus Ohm'schen Widerständen und Kondensatoren ergeben, die die frequenzabhängige Gesamtimpedanz Z ergeben.

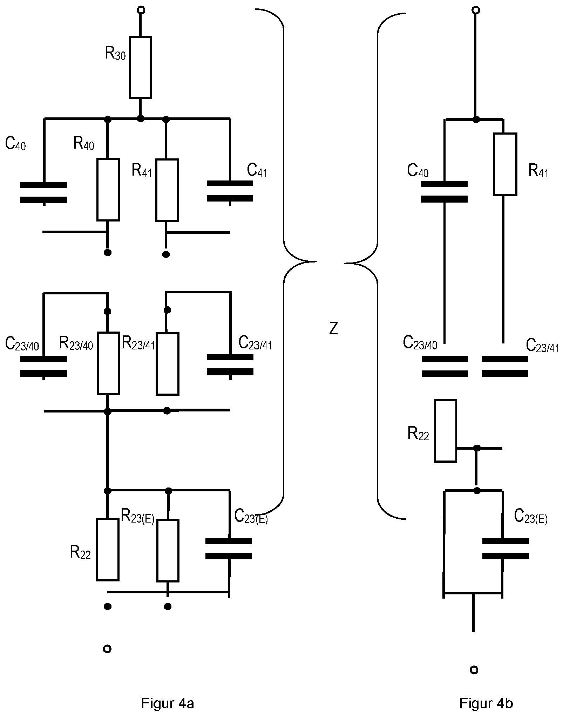

Damit lässt sich näherungsweise ein Ersatzschaltbild skizzieren, wie es in Figur 4a dargestellt ist. Darin sind:

$R_{30}$    Ohm'scher Widerstand der Hülse 30 (vergleichsweise niederohmig, liegt im m$\Omega$-Bereich)

$R_{40}$    Ohm'scher Widerstand des Klebespaltes für den Keber 40 zwischen Lichtwellenleiter 10 mit Funktionsschichtsystem 20, abhängig von der Kleberschichtdicke 40.1 $d_{K40Q}$ und der Kleberspaltlänge 40.2 $l_{K40Q}$ sowie dem spez. Widerstand $\rho_{K40}$ des Klebers 40 (dieser ist vergleichsweise hochohmig),

$C_{40}$    Kapazität des Kleberspaltes mit dem Kleber 40, abhängig von der Kleberschichtdicke 40.1 $d_{K40Q}$, der Kleberspaltlänge 40.2 $l_{K40Q}$ und dem Gesamtdurchmesser $d_{Fg}$ des Lichtwellenleiters 10 inkl. des Funktionsschichtsystems 20 $d_{Fg}$ sowie der relativen Dielektrizitätskonstante $\varepsilon_{K40}$ des Klebers 40

$R_{41}$    Ohm'scher Widerstand des Klebespaltes für den Leitkleber 41 zwischen Lichtwellenleiter 10 mit Funktionsschichtsystem 20, abhängig von der Kleberschichtdicke 41.1 $d_{K41Q}$ und der Kleberspaltlänge 41.2 $l_{K4IQ}$ sowie dem spez. Widerstand $\rho_{K41}$ des Leitklebers 41 (diese ist vergleichsweise niederohmig),

$C_{41}$    Kapazität des Kleberspaltes mit dem Leitkleber 41, abhängig von der Leitkleberschichtdicke 41.1 $d_{K41Q}$ und der Kleberspaltlänge 40.2 $l_{K41Q}$ sowie der relativen Dielektrizitätskonstante $\varepsilon_{K41}$ des Leitklebers 41

$R_{23/40}$    Ohm'scher Widerstand der Barriereschicht 23 des Funktionsschichtsystems 20 im Bereich der Verklebung mit dem Kleber 40, abhängig von seiner Barriere-Schichtdicke 23.1 $d_{23}$ und der Kleberspaltlänge 40.2 $l_{K40Q}$ sowie dem spez. Widerstand der Barriereschicht 23 $\rho_{23}$ (i.d.R. sehr hochohmig)

$C_{23/40}$    Kapazität der der Barriereschicht 23 des Funktionsschichtsystems 20 im Bereich der Verklebung mit dem Kleber 40, abhängig von seiner Barriere-Schichtdicke 23.1 $d_{23}$ und der Kleberspaltlänge 40.2 $l_{K40Q}$ sowie der relativen Dielektrizitätskonstante $\varepsilon_{23}$ der Barriereschicht 23.

$R_{23/41}$    Ohm'scher Widerstand der Barriereschicht 23 des Funktionsschichtsystems 20 im Bereich der Verklebung mit dem Leitkleber 41, abhängig von seiner Barriere-Schichtdicke 23.1 $d_{23}$ und der Kleberspaltlänge 41.2 $l_{K41Q}$ sowie dem spez. Widerstand der Barriereschicht 23 $\rho_{23}$ (i.d.R. sehr hochohmig)

$C_{23/41}$    Kapazität der der Barriereschicht 23 des Funktionsschichtsystems 20 im Bereich der Verklebung mit dem Leitkleber 41, abhängig von seiner Barriere-Schichtdicke 23.1 $d_{23}$ und der Kleberspaltlänge 41.2 $l_{K40Q}$ sowie der relativen Dielektrizitätskonstante $\varepsilon_{23}$ der Barriereschicht 23.

$R_{22}$    Ohm'scher Widerstand der Leitschicht 22, abhängig von der Leitschichtdicke $d_{22}$ und Leitschichtlänge 22.2 $l_{22}$ sowie dem spez. Widerstand der Leitschicht 22 $\rho_{22}$, welcher vergleichsweise niederohmig ist.

$R_{23(E)}$    Ohm'scher Widerstand der Barriereschicht 23 abhängig von Eintauchtiefe 22.3 E und der Barriere-Schichtdicke 23.1 $d_{23}$ und dem spez. Widerstand der Barriereschicht 23 $\rho_{23}$

$C_{23(E)}$    Kapazität der der Barriereschicht 23 des Funktionsschichtsystems 20 im Bereich der Eintauchung, abhängig von seiner Barriere-Schichtdicke 23.1 $d_{23}$ und der Eintauchtiefe 22.3 E sowie der relativen Dielektrizitätskonstante $\varepsilon_{23}$ der Barriereschicht 23.

Dieses Ersatzschaltbild in Figur 4a kann insofern vereinfacht werden, da der Ohm'sche Widerstand $R_{30}$ der Adapterhülse 30 nahezu vernachlässigbar ist, da dieser im m$\Omega$-Bereich liegt. Weiterhin ist der Ohm'scher Widerstand des Klebespaltes $R_{40}$ für den Keber 40 vergleichsweise hochohmig und trägt somit zur Gesamtimpedanz nur sehr wenig bei. Dies gilt auch für den Ohm'schen Widerstand $R_{23/40}$ der Barriereschicht 23 im Bereich des Klebers 40. Gleiches gilt auch für den Ohm'schen Widerstand $R_{23/41}$ der Barriereschicht 23 im Bereich des Leitklebers 41.

Andererseits kann der kapazitive Anteil des Kleberspaltes $C_{41}$ mit dem Leitkleber 41 gegenüber $R_{41}$ vernachlässigt werden. Ebenso kann der Ohm'scher Widerstand $R_{23(E)}$ der Barriereschicht 23 abhängig von Eintauchtiefe 22.3 E vernachlässigt werden, da dieser Anteil aufgrund des hohen Widerstands der Barriereschicht 23 sehr wenig zur Leitfähigkeit beiträgt. Daraus ergibt sich das vereinfachte Ersatzschaltbild in Figur 4b für die Gesamtimpedanz Z.

Die Einzel-Impedanzen aus dem vereinfachten Ersatzschaltbild in Figur 4b berechnen sich dabei wie folgt:

$$C_{40} = \varepsilon_0\, \varepsilon_{K40}\, \pi\, d_{Fg}\, l_{K40Q}\, /\, d_{K40Q} \tag{1}$$

$$C_{23/40} = \varepsilon_0\, \varepsilon_{23}\, \pi\, (d_F + 2\, d_L)\, l_{K40Q}\, /\, d_{23} \tag{2}$$

mit $d_F$ gleich dem Durchmesser 10.1 des Lichtwellenleiters 10 inkl. Leitschichtdicke 22.1 $d_L$ des Funktionsschichtsystems 20 und der Barriere-Schichtdicke 23.1 $d_{23}$

$$R_{41} = \rho_{K41}\, d_{K41Q}\, /\, (\pi\, l_{K41Q}\, d_{Fg}) \tag{3}$$

$$C_{23/41} = \varepsilon_0\, \varepsilon_{23}\, \pi\, (d_F + 2\, d_L)\, l_{K41Q}\, /\, d_{23} \tag{4}$$

$$R_{22} = \rho_{22}\, 4\, l_F\, /\, (((d_F + 2\, d_{22})^2 - d_F^2)\, \pi) \tag{5}$$

$$C_{23(E)} = \varepsilon_0\, \varepsilon_{23}\, \pi\, d_{Fg}\, E\, /\, d_{23} \tag{6}$$

Gemäß einem Ausführungsbeispiel ergeben sich für ein Funktionsschichtsystem 20 mit einer Leitschicht 22 aus Titan (Ti) und einer Barriereschicht 23 aus $TiO_2$ unter Berücksichtigung folgender Geometrien, Materialkonstanten und Frequenzen f folgende Einzel-Impedanzen:

| | |
|---|---|
| Erste Frequenz $f_1$: | 1.000 1/s |
| Zweite Frequenz $f_2$: | 10.000 1/s |
| Durchmesser 10.1 Lichtwellenleiter 10: | 265 $\mu$m = 2,65 E-4 m |
| Leitschichtdicke 22.1 der Ti-Leitschicht: | 0,4 $\mu$m = 0,4 E-6 m |
| Barriere-Schichtdicke 23.1 aus $TO_2$: | 100 nm = 0,1 E-6 m |
| Kleberschichtdicke 40.1 des Klebers 40: | 20 $\mu$m = 2 E-5 m |
| Kleberspaltlänge 40.2 für den Kleber 40: | 0,8 mm = 8 E-4 m |
| Kleberschichtdicke 41.1 des Leitklebers 41: | 0,1 mm = 1 E-4 m |
| Kleberspaltlänge 41.2 für den Leitklebers 41: | 1,5 mm = 1,5 E-3 m |
| Leitschichtlänge 22.2: | 50 mm = 5 E-2 m |
| $\varepsilon_0$: | 8,85 E-12 As/Vm |
| $\varepsilon_{23}$: | 180 |
| $\varepsilon_{40}$: | 10 |
| $\rho_{22}$: | 4,0 E-7 Ohm m |
| $\rho_{41}$: | 1,0 E-6 Ohm m |
| Erste Eintauchtiefe 22.3 E | 0 mm |
| | (Faserende berührt gerade die Flüssigkeit 50) |
| Zweite Eintauchtiefe 22.3 E: | 10 mm = 1 E-2 m |
| Dritte Eintauchtiefe 22.3 E: | 20 mm = 2 E-2 m |

$C_{40} = 3{,}0$ pF

$C_{23/40}$ = 11 nF
$R_{41}$ = 0,08 mΩ
$C_{23/41}$ = 20 nF
$R_{22}$ = 60 Ω
$C_{23(E=0)}$ = 0 F

Bei einer Eintauchtiefe E = 10 mm = 1 E-2 m ist

$$C_{23(E=10)} = 0,13 \text{ µF}$$

Bei einer Eintauchtiefe E = 20 mm = 2 E-2 m ist

$$C_{23(E=20)} = 0,27 \text{ µF}$$

Damit ergeben sich nachfolgende frequenzabhängige absolute Einzel-Impedanzen, wobei $XC_i$ kapazitive Blindleistungswiderstände und $ZR_i$ reelle Ohm'sche Widerstände darstellen mit

$$XC_i = 1 / \omega C_i = 1 / (2 \pi f C_i) \tag{7}$$

$$ZR_i = R_i \tag{8}$$

Für die erste Frequenz $f_1$ = 1 kHz ergeben sich folgende Werte

| | |
|---|---|
| $XC_{40}$ = | 54 MΩ |
| $XC_{23/40}$ = | 15 kΩ |
| $ZR_{41}$ = | 0,08 mΩ |
| $XC_{23/41}$ = | 8 kΩ |
| $ZR_{22}$ = | 60 Ω |
| $XC_{23(E=0 \text{ mm})}$ | extrem hochohmig |
| $XC_{23(E=10 \text{ mm})}$ | 1,2 kΩ |
| $XC_{23(E=20 \text{ mm})}$ | 600 Ω |

Für die zweite Frequenz $f_2$ = 10 kHz ergeben sich folgende Werte

| | |
|---|---|
| $XC_{40}$ = | 5,4 MΩ |
| $XC_{23/40}$ = | 1,5 kΩ |
| $ZR_{41}$ = | 0,08 mΩ |
| $XC_{23/41}$ = | 800 Ω |
| $ZR_{22}$ = | 60 Ω |
| $XC_{23(E=0 \text{ mm})}$ | extrem hochohmig |
| $XC_{23(E=10 \text{ mm})}$ | 120 Ω |
| $XC_{23(E=20 \text{ mm})}$ | 60 Ω |

Die Beispielwerte zeigen, dass bei entsprechender Frequenzwahl und Verwendung eines Leitklebers 41 im Kontaktierungsabschnitt 32 recht gute Impedanz-Verhältnisse auftreten, so dass eine Füllstandsdetektion bzw. Messung der Eindringtiefe 22.3 E mit vergleichsweise einfachem elektronischen Aufwand innerhalb der Auswerteeinheit 60 möglich ist.

Im Fall, dass das optisch elektrische Leitersystem 100 keine, insbesondere als Isolator wirkende, Barriereschicht 23 umfasst, spielen insbesondere lediglich noch Übergangswiderstände eine Rolle. Beispielsweise in einem solchen Fall, aber grundsätzlich auch unabhängig davon, lassen sich auch Gleichspannungs-Signaleinkopplungen verwenden.

Es ist dem Fachmann ersichtlich, dass die vorstehend beschriebenen Ausführungsformen beispielhaft zu verstehen sind und die Erfindung nicht auf diese beschränkt ist, sondern in vielfältiger Weise variiert werden kann, ohne den Schutzbereich der Ansprüche zu verlassen. Außerdem sind die Merkmale der optisch-elektrischen Leiteranordnung in entsprechender Weise auch als Merkmale für das Verfahren zur Herstellung einer optisch-elektrischen Leiteranordnung offenbart und umgekehrt. Merkmale definieren, unabhängig davon, ob sie in der Beschreibung, den Ansprüchen, den Figuren oder anderweitig offenbart sind, auch einzeln Bestandteile der Erfindung, selbst wenn sie zusammen mit anderen Merkmalen gemeinsam beschrieben sind.

Bezugszeichenliste

| | |
|---|---|
| 100 | Optisch-elektrisches Leitersystem |
| 1 | Optisch-elektrische Leiteranordnung |
| 10 | Lichtwellenleiter |
| 10.1 | Durchmesser |
| 11 | Optisch leitender Kern |
| 12 | Umhüllung |
| 13 | Organische Mantelschicht |
| 20 | Funktionsschichtsystem |
| 20.1 | Schichtdicke |
| 21 | Grundschichtbereich |
| 22 | Leitschicht |
| 22.1 | Leitschichtdicke |
| 22.2 | Leitschichtlänge |
| 22.3 | Eintauchtiefe |
| 23 | Barriereschicht |
| 23.1 | Barriere-Schichtdicke |
| 30 | Adapterhülse |
| 31 | Fixierungsabschnitt |
| 31.1 | Durchmesser |
| 31.2 | Länge |
| 32 | Kontaktierungsabschnitt |
| 32.1 | Durchmesser |
| 32.2 | Länge |
| 40 | Kleber |
| 40.1 | Kleberschichtdicke |
| 40.2 | Kleberspaltlänge |
| 41 | Leitkleber |
| 41.1 | Leitkleberschichtdicke |
| 41.2 | Kleberspaltlänge |
| 50 | Flüssigkeit |
| 60 | Auswerteeinheit |
| 200 | Vorrichtung zur Erfassung des Eintauchens |

**Patentansprüche**

1. Optisch-elektrisches Leitersystem (100) umfassend:

   eine optisch-elektrische Leiteranordnung (1) umfassend einen Lichtwellenleiter (10) mit einer äußeren, organischen Mantelschicht (13), und eine unmittelbar oder mittelbar auf der äußeren Mantelschicht (13) des Lichtwellenleiters (10) aufgebrachte Leitschicht (22) bestehend aus einer einzelnen Schicht oder einer Abfolge von Schichten, und
   eine zumindest bereichsweise elektrisch leitfähige Adapterhülse (30), welche die optisch-elektrische Leiteranordnung (1) mechanisch erfasst und deren Leitschicht (22) elektrisch kontaktiert, insbesondere derart, dass das optisch-elektrische Leitersystem (100) mit der Adapterhülse (30) in eine, z.B. an einem Handstück angeordnete, Anschlussfassung zur Übertragung optischer und/oder elektrischer Signale einsetzbar ist,
   wobei die Adapterhülse (30) einen elektrisch leitfähigen Kontaktierungsabschnitt (32) aufweist welcher die Leitschicht (22) der optisch-elektrischen Leiteranordnung (1) elektrisch kontaktiert, **dadurch gekennzeichnet, dass** in dem Kontaktierungsabschnitt (32) ein elektrischer Leitkleber (41) eingebracht ist, welcher die Leitschicht (22) der optisch-elektrischen Leiteranordnung (1) elektrisch kontaktiert.

2. Optisch-elektrisches Leitersystem (100) nach Anspruch 1, wobei die Adapterhülse (30)

   einen äußeren Hülsenkörper aufweist welcher eine innere Aufnahmeöffnung umschließt und wobei die Adapterhülse (30) eine Hülsenachse definiert, und
   wobei sich die optisch-elektrische Leiteranordnung (1) entlang der Hülsenachse durch die innere Aufnahmeöffnung der Adapterhülse (30) erstreckt, und
   wobei die innere Aufnahmeöffnung der Adapterhülse (30) vorzugsweise zylindrisch geformt ist und/oder konzentrisch zum äußeren Hülsenkörper angeordnet ist, und
   wobei die optisch-elektrische Leiteranordnung (1) vorzugsweise auf zumindest einer Seite der Adapterhülse (30) aus dieser herausragt und/oder derart in der Adapterhülse (30) erfasst ist, dass zumindest der Lichtwellenleiter (10) von beiden Seiten der Adapterhülse (30) zugänglich ist.

3. Optisch-elektrisches Leitersystem (100) nach einem der vorstehenden Ansprüche,

   wobei die Adapterhülse (30) einen Fixierungsabschnitt (31) aufweist welcher die optisch-elektrische Leiteranordnung (1) mechanisch erfasst und
   wobei in dem Fixierungsabschnitt (31) vorzugsweise ein Kleber (40) eingebracht ist, welcher die optisch-elektrische Leiteranordnung (1) zusätzlich fixiert und
   wobei der Kleber (40) in radialer Richtung insbesondere eine Dicke zwischen 1 und 500 Mikrometer aufweist, vorzugsweise zwischen 5 und 100 Mikrometer aufweist, besonders bevorzugt zwischen 10 und 50 Mikrometer aufweist.

4. Optisch-elektrisches Leitersystem (100) nach Anspruch 3,

   wobei der Fixierungsabschnitt (31) die optisch-elektrische Leiteranordnung (1) mit einer radialen Toleranz von weniger als $50\,\mu m$, vorzugsweise von weniger als $30\,\mu m$ erfasst, insbesondere indem der der Fixierungsabschnitt (31) gegenüber der optisch-elektrischen Leiteranordnung (1) ein radiales Spiel von weniger als $50\,\mu m$, vorzugsweise von weniger als $30\,\mu m$ aufweist und/oder
   wobei der Fixierungsabschnitt (31) die optisch-elektrische Leiteranordnung (1) derart erfasst, dass der Lichtwellenleiter (10) der optisch-elektrischen Leiteranordnung (1) zentrisch zur Hülsenachse mit einem Konzentrizitätsfehler von weniger als $50\,\mu m$, vorzugsweise von weniger als $30\,\mu m$, erfasst ist.

5. Optisch-elektrisches Leitersystem (100) nach einem der vorstehenden Ansprüche,
   wobei der Leitkleber (41) in radialer Richtung eine Dicke zwischen 1 und 500 Mikrometer aufweist, vorzugsweise zwischen 25 und 250 Mikrometer aufweist, besonders bevorzugt zwischen 50 und 150 Mikrometer aufweist.

6. Optisch-elektrisches Leitersystem (100) nach einem der Ansprüche 3 bis 5,

   wobei die innere Aufnahmeöffnung der Adapterhülse im Bereich des Fixierungsabschnitts (31) einen kleineren Querschnitt aufweist als im Bereich des Kontaktierungsabschnitts (32) und
   wobei die innere Aufnahmeöffnung vorzugsweise zwischen dem Fixierungsabschnitt (31) und dem Kontaktierungsabschnitt (32) einen Übergangsbereich von dem kleineren Querschnitt auf den größeren Querschnitt aufweist und
wobei der Übergangsbereich z.B. als Konus oder als Ringschulter ausgebildet ist.

7. Optisch-elektrisches Leitersystem (100) nach einem der vorstehenden Ansprüche,
wobei die Adapterhülse (30) ein Metall, z.B. Edelstahl oder Neusilber, oder einen leitfähigen Kunststoff oder eine leitfähige Keramik umfasst oder daraus besteht.

8. Optisch-elektrisches Leitersystem (100) nach einem der Ansprüche 1 bis 7,

wobei der ohmsche Widerstand ($R_{30}$) der Adapterhülse (30) zwischen 1 und 1000 Milliohm liegt, vorzugsweise zwischen 10 und 1000 Milliohm liegt, besonders bevorzugt zwischen 10 und 100 Milliohm liegt und/oder
wobei der ohmsche Widerstand ($R_{22}$) der Leitschicht (22) zwischen 1 und 1000 Ohm liegt, vorzugsweise zwischen 10 und 500 Ohm liegt, besonders bevorzugt zwischen 10 und 100 Ohm liegt.

9. Optisch-elektrisches Leitersystem (100) nach einem der vorstehenden Ansprüche,

wobei auf der unmittelbar oder mittelbar auf der äußeren Mantelschicht (13) des Lichtwellenleiters (10) aufgebrachten Leitschicht (22) unmittelbar oder mittelbar eine Barriereschicht (23) aufgebracht ist, wobei die Barriereschicht (23) ausgebildet ist, ein Eindiffundieren von Sauerstoff und/oder Ionen von sauren oder alkalischen Lösungen in die Leitschicht (22) zu hemmen und/oder
wobei eine oberflächliche Schicht der Leitschicht (22) zumindest eine veränderte Materialeigenschaft aufweist und eine Barriereschicht (23) bildet, wobei die Barriereschicht (23) vorzugsweise als, insbesondere natürliche, Oxidationsschicht der Leitschicht (22) und/oder als Kontaminationsfilm auf der Leitschicht (22) ausgebildet ist.

10. Optisch-elektrisches Leitersystem (100) nach einem der Ansprüche 1 bis 9,

wobei zwischen der Adapterhülse (30) und der Leitschicht (22) der optisch-elektrischen Leiteranordnung (1) eine Impedanz (Z) messbar ist und
wobei die Impedanz (Z) einen Leitschichtanteil aufweist, welcher gebildet ist durch oder zumindest abhängig ist von der Impedanz der Leitschicht (22) und/oder der Impedanz der Leitschicht (22) zu einem die optisch-elektrischen Leiteranordnung (1) umgebenden Medium (50) und
wobei die Impedanz (Z) einen Kontaktierungsanteil aufweist, welcher gebildet ist durch oder zumindest abhängig ist von dem ohmschen Widerstand ($R_{41}$) des im Kontaktierungsabschnitt (32) eingebrachten Leitklebers (41) und/oder dem kapazitiven Widerstand ($C_{41}$) des im Kontaktierungsabschnitt (32) eingebrachten Leitklebers (41) und
wobei der Kontaktierungsanteil der Impedanz (Z) kleiner gleich dem 10-fachen, vorzugsweise kleiner gleich dem 5-fachen, besonders bevorzugt kleiner gleich dem 0,5-fachen des Leitschichtanteils der Impedanz (Z) ist.

11. Optisch-elektrisches Leitersystem (100) nach Anspruch 10,

wobei der Kontaktierungsanteil der Impedanz (Z) ferner abhängig ist von dem ohmschen Widerstand ($R_{40}$) des im Fixierungsabschnitt (31) eingebrachten Klebers (40) und/oder dem kapazitiven Widerstand ($C_{40}$) des im Fixierungsabschnitt (31) eingebrachten Klebers (40), und/oder
wobei der Kontaktierungsanteil der Impedanz (Z) ferner abhängig ist von dem ohmschen Widerstand ($R_{23/40}$) der Barriereschicht (23) im Bereich des im Fixierungsabschnitt (31) eingebrachten Klebers (40) und/oder dem kapazitiven Widerstand ($C_{23/40}$) der Barriereschicht (23) im Bereich des im Fixierungsabschnitt (31) eingebrachten Klebers (40).

12. Optisch-elektrisches Leitersystem (100) nach einem der Ansprüche 10 oder 11, wobei die zwischen der Adapterhülse (30) und der Leitschicht (22) der optisch-elektrischen Leiteranordnung (1) messbare Impedanz (Z)

zwischen dem äußeren Hülsenkörper der Adapterhülse (30) und einem Bereich der Leitschicht (22) in einem aus der Adapterhülse (30) herausragenden, vorzugsweise endseitigem, Abschnitt der optisch-elektrischen Leiteranordnung (1) messbar ist und/oder
bei einer bestimmten Auswertefrequenz oder über einen Auswertefrequenzbereich messbar ist, wobei die Auswertefrequenz oder der Auswertefrequenzbereich vorzugsweise im Bereich von 1 Hz bis 10 GHz, bevorzugter im Bereich von 10 Hz bis 2,4 GHz, nochmals bevorzugter im Bereich von 100 Hz bis 1 MHz, besonders bevorzugt im Bereich von 1 kHz bis 100 kHz und ganz besonders bevorzugt im Bereich von 1 KHz bis 10 kHz liegt.

13. Optisch-elektrisches Leitersystem (100) nach einem der vorstehenden Ansprüche, wobei die optisch-elektrische Leiteranordnung (1) umfasst:

ein auf der äußeren Mantelschicht (13) des Lichtwellenleiters (10) befindliches Funktionsschichtsystem (20) mit einem Grundschichtbereich (21) bestehend aus einer einzelnen Schicht oder einer Abfolge von Schichten und der elektrisch leitenden Leitschicht (22) bestehend aus einer einzelnen Schicht oder einer Abfolge von Schichten, wobei die Leitschicht (22) auf dem Grundschichtbereich (21) aufgebracht ist und
wobei der Lichtwellenleiter (10) vorzugsweise umfasst:

- einen optisch leitenden Kern (11) und
- vorzugsweise eine Umhüllung (12), welche den Kern (11) umgibt und zwischen dem Kern (11) und der äußeren Mantelschicht (13) angeordnet ist,
- und wobei der optisch leitende Kern (11) vorzugsweise einen Brechungsindex aufweist, welcher größer ist als ein Brechungsindex, welchen die äußere Mantelschicht (13) aufweist und/oder größer ist als ein Brechungsindex, welchen die Umhüllung (12) aufweist.

14. Optisch-elektrisches Leitersystem (100) nach einem der vorstehenden Ansprüche, wobei die organische Mantelschicht (13) Polyamid, PA, Polyimid, PI, oder Polymethylmethacrylat, PMMA, oder Wachs, wachsähnliche Bestandteile oder Alkylsilan umfasst.

15. Optisch-elektrisches Leitersystem (100) nach einem der Ansprüche 13 bis 14, mit zumindest einem der folgenden Merkmale a)-d):

a) wobei der Grundschichtbereich (21) umfasst:

- eine auf der äußeren Mantelschicht (13) aufgebrachte Schicht mit einem Oxid, insbesondere $SiO_2$, $TiO_2$, $Al_2O_3$, $SnO_2$, $HfO_2$ oder einem Borid, Carbid, Nitrid, Oxinitrid, Carbonitrid oder einem Metall, insbesondere Si, Ti, Mo oder Cr,
- oder eine auf der äußeren Mantelschicht (13) aufgebrachte Abfolge solcher Schichten

b) wobei der Grundschichtbereich (21) umfasst oder gebildet ist durch eine oberflächliche Schicht der äußeren Mantelschicht (13) mit zumindest einer veränderten Oberflächeneigenschaft, insbesondere einer erhöhten Oberflächenenergie und/oder einer erhöhten Anzahl von Sauerstoffradikalen, insbesondere hergestellt oder herstellbar mittels eines chemischen und/oder physikalischen Prozesses zur Veränderung zumindest einer Oberflächeneigenschaft der äußeren Mantelschicht (13),
c) wobei der Grundschichtbereich eine Dicke zwischen 5nm und 3000nm, bevorzugt zwischen 5nm und 1000nm, besonders bevorzugt zwischen 10nm und 100nm aufweist und/oder wobei die einzelnen Schichten des Grundschichtbereichs jeweils eine Dicke zwischen 5nm und 1000nm bevorzugt zwischen 10nm und 100nm aufweisen,
d) wobei zwischen dem Grundschichtbereich (21) und der darauf aufgebrachten Leitschicht (22) eine Haftung besteht, welche größer ist als eine Haftung, welche zwischen der äußeren Mantelschicht (13) und einer darauf aufgebrachten identischen Leitschicht (22) bestehen würde.

16. Optisch-elektrisches Leitersystem (100) nach einem der Ansprüche 13 bis 15, wobei zumindest eine der auf der äußeren Mantelschicht (13) aufgebrachten Schichten des Grundschichtbereichs (21) ausgebildet ist, ein Eindiffundieren von Sauerstoff und/oder Ionen von sauren oder alkalischen Lösungen in die Leitschicht (22) zu hemmen.

17. Optisch-elektrisches Leitersystem (100) nach einem der Ansprüche 13 bis 16,

wobei der Grundschichtbereich (21) einen thermischen Ausdehnungskoeffizienten aufweist, welcher zwischen einem thermischen Ausdehnungskoeffizienten liegt, welchen die Mantelschicht (13) aufweist, und einem thermischen Ausdehnungskoeffizienten, welchen die Leitschicht (22) aufweist und/oder
wobei der Grundschichtbereich eine Abfolge von Schichten umfasst, welche jeweils einen thermischen Ausdehnungskoeffizienten aufweisen, welche entsprechend der Abfolge der Schichten aufsteigen oder absteigen.

18. Optisch-elektrisches Leitersystem (100) nach einem der vorstehenden Ansprüche, mit zumindest einem der Merkmale a)-c):

a) wobei die Leitschicht (22) umfasst:

- eine auf dem Grundschichtbereich (21) aufgebrachte Schicht mit Titan, Silizium, Aluminium, Gold, Silber, Molybdän, Wolfram oder Zirkon, insbesondere einer Legierung eines dieser Stoffe mit Ni, Zn, Y, Sn, Ge
- oder eine auf dem Grundschichtbereich (21) aufgebrachte Abfolge solcher Schichten,

b) wobei die Leitschicht (22) eine Dicke zwischen 5nm und 6000nm, bevorzugt zwischen 5nm und 2000nm, besonders bevorzugt zwischen 10nm und 200nm aufweist und/oder wobei einzelne Schichten der Leitschicht jeweils eine Dicke zwischen 5nm und 2000nm bevorzugt zwischen 10nm und 200nm aufweisen,
c) wobei die Leitschicht (22) einen Flächenwiderstand zwischen 0,01 und 1000 Ohm/sqr, bevorzugt zwischen 0,01 und 100 Ohm/sqr, besonders bevorzugt zwischen 0,01 und 50 Ohm/sqr aufweist.

19. Optisch-elektrisches Leitersystem (100) nach einem der Ansprüche 13 bis 18, wobei das Funktionsschichtsystem (20) ferner umfasst:

- eine auf der Leitschicht (22) aufgebrachte Barriereschicht (23) bestehend aus einer einzelnen Schicht oder einer Abfolge von Schichten, wobei die Barriereschicht (23) ausgebildet ist, ein Eindiffundieren von Sauerstoff und/oder Ionen von sauren oder alkalischen Lösungen in die Leitschicht (22) zu hemmen und/oder
- eine Barriereschicht (23) ausgebildet als oberflächliche Schicht der Leitschicht (22) mit zumindest einer veränderten Materialeigenschaft und/oder ausgebildet als, insbesondere natürliche, Oxidationsschicht der Leitschicht (22) und/oder als Kontaminationsfilm auf der Leitschicht (22).

20. Optisch-elektrisches Leitersystem (100) nach Anspruch 19, wobei zumindest eine der auf der Leitschicht (22) aufgebrachten Schichten der Barriereschicht (23) eine Härte von mindestens 800 HV, vorzugsweise mindestens 1200 HV, besonders bevorzugt mindestens 2000 HV gemäß der Prüfnorm DIN EN ISO 14577-4:2007-8 aufweist.

21. Optisch-elektrisches Leitersystem (100) nach Anspruch 19 oder 20, wobei die Barriereschicht (23) umfasst:

- eine auf der Leitschicht (22) aufgebrachte Schicht mit einem Nitrid, insbesondere $Si_3N_4$, BN, AIN, TiN, AlSiN, SiON, SiAlON oder einer Legierung eines dieser Stoffe, oder mit einem Oxid, insbesondere Oxide von Si, Al, Ti, Zr, Zn, Sn, Ta, Nb, Y, $TiO_2$, $SiO_2$ oder einem ternären System aus zumindest einem dieser Stoffe, oder mit einem Carbid, Borid, Oxinitrid, Carbonitrid,
- oder eine auf der Leitschicht (22) aufgebrachte Abfolge solcher Schichten.

22. Vorrichtung (200) zur Erfassung des Eintauchens einer optisch-elektrischen Leiteranordnung (1) in ein leitfähiges Medium (50), insbesondere innerhalb eines tierischen oder menschlichen Körpers, umfassend:

ein optisch-elektrisches Leitersystem (100), nach einem der Ansprüche 1 bis 21,
eine Auswerteeinheit (60) welche mit der zumindest bereichsweise elektrisch leitfähigen Adapterhülse (30) und dem leitfähigen Medium (50) elektrisch verbindbar oder verbunden ist, und eingerichtet ist, eine Impedanz oder Impedanzänderung zwischen der Adapterhülse (30) und dem Medium (50) zu bestimmen, wenn die optisch-elektrische Leiteranordnung (1) in das leitfähige Medium (50) eingetaucht ist oder wird, derart, dass ein Eintauchen der optisch-elektrischen Leiteranordnung (1) in das Medium (50) erfasst werden kann und/oder eine Eintauchtiefe der optisch-elektrischen Leiteranordnung (1) in das Medium (50) bestimmt werden kann.

23. Verfahren zur Herstellung eines optisch-elektrischen Leitersystems (100), nach einem der Ansprüche 1 bis 21, umfassend:

Bereitstellen oder Herstellen der zumindest bereichsweise elektrisch leitfähigen Adapterhülse (30),
Bereitstellen oder Herstellen der optisch-elektrischen Leiteranordnung (1),
Einführen der optisch-elektrischen Leiteranordnung (1) in die Adapterhülse (30) derart, dass die Adapterhülse (30) die optisch-elektrische Leiteranordnung (1) mechanisch erfasst und deren Leitschicht (22) elektrisch kontaktiert,
wobei die bereitgestellte oder hergestellte Adapterhülse (30) vorzugsweise einen Fixierungsabschnitt (31) aufweist, um die optisch-elektrische Leiteranordnung (1) mechanisch zu erfassen und
wobei in den Fixierungsabschnitt (31) vorzugsweise ein Kleber (40) eingebracht wird, um die optisch-elektrische Leiteranordnung (1) zusätzlich zu fixieren, und
Einbringen des elektrischen Leitklebers (41) in den Kontaktierungsabschnitt (32), um die Leitschicht (22) der optisch-elektrischen Leiteranordnung (1) elektrisch zu kontaktieren.

24. Verfahren nach Anspruch 23, wobei das Herstellen der optisch-elektrischen Leiteranordnung (1) umfasst:

- Bereitstellen des Lichtwellenleiters (10) mit der äußeren Mantelschicht (13),
- Beschichten der Mantelschicht (13) des Lichtwellenleiters mit einem Funktionsschichtsystem (20), umfassend:

- Erzeugen eines Grundschichtbereichs (21) bestehend aus einer einzelnen Schicht oder einer Abfolge von Schichten,
- Auf dem Grundschichtbereich Aufbringen der elektrisch leitenden Leitschicht (22) bestehend aus einer einzelnen Schicht oder einer Abfolge von Schichten.

25. Verfahren nach Anspruch 24, wobei das Erzeugen des Grundschichtbereichs (21) umfasst:

- Vorbehandeln der Mantelschicht (13) des Lichtwellenleiters (10), insbesondere mittels chemischer oder physikalischer Prozesse zur Veränderung der Oberflächeneigenschaften der Mantelschicht (13), um eine oberflächliche Schicht mit zumindest einer veränderten Oberflächeneigenschaft zu erzeugen, insbesondere eine oberflächliche Schicht mit einer erhöhten Oberflächenenergie und/oder einer erhöhten Anzahl von Sauerstoffradikalen, und/oder um Rückstände zu entfernen.

26. Verfahren nach einem der Ansprüche 23 bis 25,

wobei das Aufbringen zumindest zweier Schichten im Vakuum und ohne Vakuumbruch erfolgt und/oder wobei das Aufbringen einer oder mehrerer Schichten unter Zugabe von Sauerstoff und Argon erfolgt, wobei mit dem Verhältnis von Sauerstoff zum Gesamtfluss Sauerstoff und Argon der metallische bzw. dielektrische Charakter der jeweiligen Schicht beeinflusst wird.

## Claims

1. An opto-electrical conductor system (100) comprising:

an opto-electrical conductor assembly (1) comprising an optical waveguide (10) having an outer organic jacket layer (13), and a conductive layer (22) applied directly or indirectly over the outer jacket layer (13) of the optical waveguide (10) and consisting of a single layer or a sequence of layers; and an adapter sleeve (30) that is electrically conductive at least in sections thereof, and which mechanically retains the opto-electrical conductor assembly (1) and makes electrical contact with the conductive layer (22) thereof, in particular in such a manner that the opto-electrical conductor system (100) including the adapter sleeve (30) can be fitted into a connection socket for transfer of optical and/or electrical signals, which socket is provided in a handpiece, for example; wherein the adapter sleeve (30) comprises an electrically conductive contacting portion (32) which electrically contacts the conductive layer (22) of the opto-electrical conductor assembly (1);

**characterized in that**
the contacting portion (32) comprises an electrically conductive adhesive (41) introduced therein, which electrically contacts the conductive layer (22) of the opto-electrical conductor assembly (1).

2. The opto-electrical conductor system (100) according to claim 1,

wherein the adapter sleeve (30) has an outer sleeve body which encloses an inner receiving opening, and wherein the adapter sleeve (30) defines a sleeve axis; and wherein the opto-electrical conductor assembly (1) extends along the sleeve axis through said inner receiving opening of the adapter sleeve (30); and wherein the inner receiving opening of the adapter sleeve (30) is preferably cylindrical in shape and/or is arranged concentrically to the outer sleeve body; and wherein the opto-electrical conductor assembly (1) projects out of the adapter sleeve (30) preferably on at least one end of the adapter sleeve (30) and/or is retained in the adapter sleeve (30) such that at least the optical waveguide (10) is accessible from both ends of the adapter sleeve (30).

**3.** The opto-electrical conductor system (100) according to any one of the preceding claims,

wherein the adapter sleeve (30) comprises a fixing portion (31) which mechanically retains the optical-electrical conductor assembly (1); and

wherein the fixing portion (31) preferably comprises an adhesive (40) introduced therein, which additionally fixes the opto-electrical conductor assembly (1); and

wherein the adhesive (40) has a thickness in the radial direction of between 1 and 500 micrometres, preferably between 5 and 100 micrometres, most preferably between 10 and 50 micrometres.

**4.** The opto-electrical conductor system (100) according to claim 3,

wherein the fixing portion (31) retains the opto-electrical conductor assembly (1) with a radial tolerance of less than 50 μm, preferably less than 30 μm, in particular by providing the fixing portion (31) with a radial allowance of less than 50 μm, preferably less than 30 μm relative to the opto-electrical conductor assembly (1); and/or

wherein the fixing portion (31) retains the opto-electrical conductor assembly (1) such that the optical waveguide (10) of the opto-electrical conductor assembly (1) is retained in a centred manner relative to the sleeve axis, with a concentricity error of less than 50 μm, preferably less than 30 μm.

**5.** The opto-electrical conductor system (100) according to any one of the preceding claims,

wherein the conductive adhesive (41) has a thickness in the radial direction between 1 and 500 micrometres, preferably between 25 and 250 micrometres, most preferably between 50 and 150 micrometres.

**6.** The opto-electrical conductor system (100) according to any one of claims 3 to 5,

wherein the inner receiving opening of the adapter sleeve has a smaller cross section within the range of the fixing portion (31) than within the range of the contacting portion (32); and

wherein the inner receiving opening preferably has a transition area between the fixing portion (31) and the contacting portion (32) from the smaller cross section to the larger cross section; and

wherein the transition area is conical or in the form an annular shoulder, for example.

**7.** The opto-electrical conductor system (100) according to any one of the preceding claims,

wherein the adapter sleeve (30) comprises or is made of a metal, e.g. stainless steel or nickel silver, or a conductive plastics material or a conductive ceramic.

**8.** The opto-electrical conductor system (100) according to any one of claims 1 to 7,

wherein the ohmic resistance ($R_{30}$) of the adapter sleeve (30) is between 1 and 1000 milliohms, preferably between 10 and 1000 milliohms, most preferably between 10 and 100 milliohms; and/or

wherein the ohmic resistance ($R_{22}$) of the conductive layer (22) is between 1 and 1000 ohms, preferably between 10 and 500 ohms, most preferably between 10 and 100 ohms.

**9.** The opto-electrical conductor system (100) according to any one of the preceding claims,

wherein a barrier layer (23) is disposed directly or indirectly over the conductive layer (22) that is disposed directly or indirectly over the outer jacket layer (13) of the optical waveguide (10), wherein said barrier layer (23) is formed to inhibit oxygen and/or ions of acidic or alkaline solutions from diffusing into the conductive layer (22); and/or

wherein a near-surface layer of the conductive layer (22) has at least one altered material property and forms a barrier layer (23), wherein the barrier layer (23) is preferably in the form of an in particular natural oxidation layer of the conductive layer (22) and/or a contamination film on the conductive layer (22).

**10.** The opto-electrical conductor system (100) according to any one of claims 1 to 9,

wherein an impedance (Z) can be measured between the adapter sleeve (30) and the conductive layer (22) of the opto-electrical conductor assembly (1); and

wherein said impedance (Z) has a conductive layer component which is defined by or at least depends on the impedance of the conductive layer (22) and/or the impedance of the conductive layer (22) to a medium (50) surrounding the opto-electrical conductor assembly (1); and

wherein the impedance (Z) has a contact component which is defined by or at least depends on the ohmic resistance ($R_{41}$) of the conductive adhesive (41) introduced in the contacting portion (32) and/or the capacitive resistance ($C_{41}$) of the conductive adhesive (41) introduced in the contacting portion (32); and

wherein the contact component of the impedance (Z) is less than or equal to 10 times, preferably less than or equal to 5 times, most preferably less than or equal to 0.5 times the conductive layer component of the impedance (Z).

11. The opto-electrical conductor system (100) according to claim 10,

wherein the contact component of the impedance (Z) also depends on the ohmic resistance ($R_{40}$) of the adhesive (40) introduced in the fixing portion (31) and/or the capacitive resistance ($C_{40}$) of the adhesive (40) introduced in the fixing portion (31); and/or

wherein the contact component of the impedance (Z) also depends on the ohmic resistance ($R_{23/40}$) of the barrier layer (23) within the range of the adhesive (40) introduced in the fixing portion (31) and/or the capacitive resistance ($C_{23/40}$) of the barrier layer (23) within the range of the adhesive (40) introduced in the fixing portion (31).

12. The opto-electrical conductor system (100) according to any one of claims 10 or 11,
wherein the impedance (Z) that can be measured between the adapter sleeve (30) and the conductive layer (22) of the opto-electrical conductor assembly (1)

can be measured between the outer sleeve body of the adapter sleeve (30) and an area of the conductive layer (22) in a portion of the opto-electrical conductor assembly (1) protruding from the adapter sleeve (30), preferably at the end thereof; and/or

can be measured at a specific evaluation frequency or over an evaluation frequency range, the evaluation frequency or the evaluation frequency range preferably being in the range from 1 Hz to 10 GHz, more preferably in the range from 10 Hz to 2.4 GHz, yet more preferably in the range from 100 Hz to 1 MHz, even more preferably in the range from 1 kHz to 100 kHz and most preferably in the range from 1 kHz to 10 kHz.

13. The opto-electrical conductor system (100) according to any one of the preceding claims,

wherein the opto-electrical conductor assembly (1) comprises:
a functional layer system (20) disposed on the outer jacket layer (13) of the optical waveguide (10), comprising a base layer portion (21) consisting of a single layer or a sequence of layers, and the electrically conductive layer (22) consisting of a single layer or a sequence of layers, wherein the conductive layer (22) is disposed over the base layer portion (21); and
wherein the optical waveguide (10) preferably comprises:

- an optically conducting core (11); and
- preferably a cladding (12) surrounding the core (11) and arranged between the core (11) and the outer jacket layer (13);
- and wherein the optically conducting core (11) preferably has a refractive index that is greater than a refractive index of the outer jacket layer (13) and/or greater than a refractive index of said cladding (12).

14. The opto-electrical conductor system (100) according to any one of the preceding claims, wherein the organic jacket layer (13) comprises polyamide, PA, polyimide, PI, or polymethyl methacrylate, PMMA, wax, wax-like components or alkylsilane.

15. The opto-electrical conductor system (100) according to any one of claims 13 to 14, comprising at least one of the following features a) - d):

a) wherein the base layer portion (21) comprises:

- a layer disposed on the outer jacket layer (13), which layer includes an oxide, in particular $SiO_2$, $TiO_2$, $Al_2O_3$, $SnO_2$, $HfO_2$, or a boride, carbide, nitride, oxynitride, carbonitride, or a metal, in particular Si, Ti, Mo or Cr;
- or a sequence of such layers disposed on the outer jacket layer (13);

b) wherein the base layer portion (21) comprises or consists of a near-surface layer of the outer jacket layer (13) which has at least one altered surface property, in particular increased surface energy and/or an increased number of oxygen radicals, in particular produced or producible by a chemical and/or physical process for altering at least one surface property of the outer jacket layer (13);

c) wherein the base layer portion has a thickness between 5 nm and 3000 nm, preferably between 5 nm and 1000 nm, most preferably between 10 nm and 100 nm, and/or wherein the individual layers of the base layer portion each have a thickness between 5 nm and 1000 nm, preferably between 10 nm and 100 nm;

d) wherein an adhesion between the base layer portion (21) and the conductive layer (22) disposed thereon is greater than an adhesion that would exist between the outer jacket layer (13) and an identical conductive layer (22) applied thereto.

16. The opto-electrical conductor system (100) according to any one of claims 13 to 15, wherein at least one of the layers of the base layer portion (21) disposed over the outer jacket layer (13) is formed to inhibit oxygen and/or ions of acidic or alkaline solutions from diffusing into the conductive layer (22).

17. The opto-electrical conductor system (100) according to any one of claims 13 to 16,

wherein the base layer portion (21) has a thermal expansion coefficient ranging between a thermal expansion coefficient of the jacket layer (13) and a thermal expansion coefficient of the conductive layer (22); and/or wherein the base layer portion comprises a sequence of layers having a thermal expansion coefficient which increases or decreases across the sequence of layers.

18. The opto-electrical conductor system (100) according to any one of the preceding claims, comprising at least one of the features a) - c):

a) wherein the conductive layer (22) comprises:

- a layer disposed on the base layer portion (21) comprising titanium, silicon, aluminium, gold, silver, molybdenum, tungsten or zirconium, in particular an alloy of any of these substances with Ni, Zn, Y, Sn, Ge;
- or a sequence of such layers disposed on the base layer portion (21);

b) wherein the conductive layer (22) has a thickness between 5 nm and 6000 nm, preferably between 5 nm and 2000 nm, most preferably between 10 nm and 200 nm, and/or wherein individual layers of the conductive layer each have a thickness between 5 nm and 2000 nm, preferably between 10 nm and 200 nm;

c) wherein the conductive layer (22) has a surface resistance between 0.01 and 1000 ohms/sqr, preferably between 0.01 and 100 ohms/sqr, most preferably between 0.01 and 50 ohms/sqr.

19. The opto-electrical conductor system (100) according to any one of claims 13 to 18, wherein the functional layer system (20) furthermore comprises:

- a barrier layer (23) disposed on the conductive layer (22) and consisting of a single layer or a sequence of layers, wherein the barrier layer (23) is formed to inhibit oxygen and/or ions of acidic or alkaline solutions from diffusing into the conductive layer (22); and/or
- a barrier layer (23) in the form of a near-surface layer of the conductive layer (22) and having at least one altered material property, and/or in the form of an in particular natural oxidation layer of the conductive layer (22) and/or as a contamination film on the conductive layer (22).

20. The opto-electrical conductor system (100) according to claim 19, wherein at least one of the layers of the barrier layer (23) disposed over the conductive layer (22) has a hardness of at least 800 HV, preferably at least 1200 HV, most preferably at least 2000 HV, according to the DIN EN ISO 14577-4:2007-8 test standard.

21. The opto-electrical conductor system (100) according to claim 19 or 20, wherein the barrier layer (23) comprises:

- a layer disposed over the conductive layer (22) and comprising a nitride, in particular $Si_3N_4$, BN, AlN, TiN, AlSiN, SiON, SiAlON or an alloy of any of these substances, or an oxide, in particular oxides of Si, Al, Ti, Zr, Zn, Sn, Ta, Nb, Y, $TiO_2$, $SiO_2$ or a ternary system including at least one of these substances, or a carbide, boride, oxynitride, carbonitride;
- or a sequence of such layers disposed over the conductive layer (22).

22. A device (200) for detecting the immersion of an opto-electrical conductor assembly (1) in a conductive medium (50), in particular within an animal or human body, comprising:

an opto-electrical conductor system (100) according to any one of claims 1 to 21;
an evaluation unit (60) which is electrically connectible or connected to the adapter sleeve (30) that is electrically conductive at least in sections thereof and to the conductive medium (50), and is adapted to determine an impedance or impedance change between the adapter sleeve (30) and the medium (50) when the opto-electrical conductor assembly (1) is or is being immersed in the conductive medium (50), such that an immersion of the opto-electrical conductor assembly (1) in the medium (50) can be detected and/or an immersion depth of the opto-electrical conductor assembly (1) in the medium (50) can be determined.

23. A method for producing an opto-electrical conductor system (100) according to any one of claims 1 to 21, comprising:

providing or producing the at least partially electrically conductive adapter sleeve (30);
providing or producing the opto-electrical conductor assembly (1);
inserting the opto-electrical conductor assembly (1) into the adapter sleeve (30) in such a way that the adapter sleeve (30) mechanically retains the opto-electrical conductor assembly (1) and makes electrical contact with the conductive layer (22) thereof;
wherein the provided or produced adapter sleeve (30) preferably comprises a fixing portion (31) for mechanically retaining the opto-electrical conductor assembly (1); and
wherein preferably an adhesive (40) is introduced into the fixing portion (31) for additionally fixing the opto-electrical conductor assembly (1); and
introducing the electrically conductive adhesive (41) into the contacting portion (32) in order to establish electrical contact to the conductive layer (22) of the opto-electrical conductor assembly (1).

24. The method according to claim 23, wherein the producing of the opto-electrical conductor assembly (1) comprises:

- providing the optical waveguide (10) with the outer jacket layer (13);
- coating the jacket layer (13) of the optical waveguide with a functional layer system (20), comprising:

  - producing a base layer portion (21) consisting of a single layer or a sequence of layers;
  - depositing, onto the base layer portion, the electrically conductive layer (22) consisting of a single layer or a sequence of layers.

25. The method according to claim 24, wherein the producing of the base layer portion (21) comprises:

- pre-treating the jacket layer (13) of the optical waveguide (10), in particular by chemical or physical processes so as to alter the surface properties of the jacket layer (13) to produce a near-surface layer having at least one altered surface property, in particular a near-surface layer with an increased surface energy and/or an increased number of oxygen radicals, and/or to remove residues.

26. The method according to any one of claims 23 to 25,

wherein at least two layers are deposited in a vacuum and without interrupting the vacuum; and/or
wherein one or more layers are deposited while introducing oxygen and argon, wherein the ratio of oxygen to the total flow of oxygen and argon is used to control the metallic or dielectric nature of the respective layer.


**Revendications**

1. Système de conducteur optique-électrique (100), comprenant :

un dispositif conducteur optique-électrique (1) comportant un guide d'ondes optiques (10), doté d'une couche de gainage extérieure (13) organique, et une couche conductrice (22) qui est appliquée directement ou indirectement sur la couche de gainage extérieure (13) du guide d'ondes optiques (10) et est constituée d'une couche individuelle ou d'une succession de couches, et
un manchon adaptateur (30) qui est conducteur électrique au moins par portions et qui saisit mécaniquement le dispositif conducteur optique-électrique (1) et établit un contact électrique avec la couche conductrice (22) de celui-ci, notamment de manière à ce que le système de conducteur optique-électrique (100) puisse être inséré avec le manchon adaptateur (30) dans un support de raccordement disposé par exemple sur une poignée, en vue de la transmission de signaux optiques et/ou électriques,

dans lequel le manchon adaptateur (30) présente une partie de contact (32) électriquement conductrice qui établit un contact électrique avec la couche conductrice (22) du dispositif conducteur optique-électrique (1), **caractérisé en ce que** dans la partie de contact (32), est introduit un adhésif conducteur (41) électrique qui établit un contact électrique avec la couche conductrice (22) du dispositif conducteur optique-électrique (1).

2. Système de conducteur optique-électrique (100) selon la revendication 1, dans lequel le manchon adaptateur (30)

présente un corps de manchon extérieur qui entoure une ouverture d'accueil interne, et le manchon adaptateur (30) définit un axe de manchon, et

dans lequel le dispositif conducteur optique-électrique (1) s'étend le long de l'axe de manchon, à travers l'ouverture d'accueil interne du manchon adaptateur (30), et

dans lequel l'ouverture d'accueil interne du manchon adaptateur (30) a de préférence une forme cylindrique et/ou est disposée de façon concentrique avec le corps de manchon externe, et

dans lequel le dispositif conducteur optique-électrique (1) dépasse du manchon adaptateur (30), de préférence sur au moins un côté de celui-ci, et/ou est saisi dans le manchon adaptateur (30) de manière à ce qu'au moins le guide d'ondes optiques (10) soit accessible depuis les deux côtés du manchon adaptateur (30).

3. Système de conducteur optique-électrique (100) selon l'une des revendications précédentes,

dans lequel le manchon adaptateur (30) présente une partie de fixation (31) qui saisit mécaniquement le dispositif conducteur optique-électrique (1), et

dans lequel un adhésif (40) est de préférence introduit dans la partie de fixation (31) et assure une fixation supplémentaire du dispositif conducteur optique-électrique (1), et

dans lequel l'adhésif (40) présente, dans la direction radiale, notamment une épaisseur comprise entre 1 et 500 micromètres, de préférence entre 5 et 100 micromètres, et de manière particulièrement avantageuse entre 10 et 50 micromètres.

4. Système de conducteur optique-électrique (100) selon la revendication 3,

dans lequel la partie de fixation (31) saisit le dispositif conducteur optique-électrique (1) avec une tolérance radiale de moins de 50 $\mu$m, de préférence de moins de 30 $\mu$m, notamment par le fait que la partie de fixation (31) présente un jeu radial de moins de 50 $\mu$m, de préférence de moins de 30 $\mu$m, par rapport au dispositif conducteur optique-électrique (1), et/ou

dans lequel la partie de fixation (31) saisit le dispositif conducteur optique-électrique (1) de manière à ce que le guide d'ondes optiques (10) du dispositif conducteur optique-électrique (1) soit saisi de façon centrée par rapport à l'axe de manchon, avec une erreur de concentricité de moins de 50 $\mu$m, de préférence de moins de 30 $\mu$m.

5. Système de conducteur optique-électrique (100) selon l'une des revendications précédentes, dans lequel l'adhésif conducteur (41) présente dans la direction radiale une épaisseur comprise entre 1 et 500 micromètres, de préférence entre 25 et 250 micromètres, et de manière particulièrement avantageuse entre 50 et 150 micromètres.

6. Système de conducteur optique-électrique (100) selon l'une des revendications 3 à 5,

dans lequel l'ouverture d'accueil interne du manchon adaptateur présente dans la région de la partie de fixation (31) une section transversale qui est plus petite que dans la région de la partie de contact (32), et

dans lequel l'ouverture d'accueil interne présente, de préférence entre la partie de fixation (31) et la partie de contact (32), une zone de transition de la section transversale plus petite vers la section plus grande, et dans lequel la zone de transition est réalisée par exemple sous forme de cône ou d'épaulement annulaire.

7. Système de conducteur optique-électrique (100) selon l'une des revendications précédentes, dans lequel le manchon adaptateur (30) comprend un métal, par exemple de l'acier inoxydable ou du maillechort, ou une matière plastique conductrice ou une céramique conductrice, ou est constitué de ces matériaux.

**8.** Système de conducteur optique-électrique (100) selon l'une des revendications 1 à 7,

dans lequel la résistance ohmique ($R_{30}$) du manchon adaptateur (30) est comprise entre 1 et 1 000 milliohms, de préférence entre 10 et 1 000 milliohms, et de manière particulièrement avantageuse entre 10 et 100 milliohms, et/ou

dans lequel la résistance ohmique ($R_{22}$) de la couche conductrice (22) est comprise entre 1 et 1 000 ohms, de préférence entre 10 et 500 ohms, et de manière particulièrement avantageuse entre 10 et 100 ohms.

**9.** Système de conducteur optique-électrique (100) selon l'une des revendications précédentes,

dans lequel une couche barrière (23) est appliquée directement ou indirectement sur la couche conductrice (22) appliquée directement ou indirectement sur la couche de gainage extérieure (13) du guide d'ondes optiques (10), la couche barrière (23) étant réalisée de manière à freiner la diffusion d'oxygène et/ou d'ions de solutions acides ou alcalines dans la couche conductrice (22), et/ou

dans lequel une couche superficielle de la couche conductrice (22) présente au moins une propriété de matériau modifiée et forme une couche barrière (23), la couche barrière (23) étant réalisée de préférence comme couche d'oxydation, notamment naturelle, de la couche conductrice (22) et/ou comme film de contamination sur la couche conductrice (22).

**10.** Système de conducteur optique-électrique (100) selon l'une des revendications 1 à 9,

dans lequel une impédance (Z) peut être mesurée entre le manchon adaptateur (30) et la couche conductrice (22) du dispositif conducteur optique-électrique (1), et

dans lequel l'impédance (Z) présente une part de couche conductrice qui est constituée de l'impédance de la couche conductrice (22) ou dépend au moins de celle-ci et/ou de l'impédance de la couche conductrice (22) par rapport à un milieu (50) entourant le dispositif conducteur optique-électrique (1), et

dans lequel l'impédance (Z) présente une part de contact qui est constituée de la résistance ohmique ($R_{41}$) de l'adhésif conducteur (41) introduit dans la partie de contact (32) ou dépend au moins de celle-ci et/ou de la résistance capacitive ($C_{41}$) de l'adhésif conducteur (41) introduit dans la partie de contact (32), et

dans lequel la part de contact de l'impédance (Z) est inférieure ou égale à 10 fois, de préférence inférieure ou égale à 5 fois, et de façon particulièrement avantageuse inférieure ou égale à 0,5 fois la part de couche conductrice de l'impédance (Z).

**11.** Système de conducteur optique-électrique (100) selon la revendication 10,

dans lequel la part de contact de l'impédance (Z) dépend en outre de la résistance ohmique ($R_{40}$) de l'adhésif (40) introduit dans la partie de fixation (31) et/ou de la résistance capacitive ($C_{40}$) de l'adhésif (40) introduit dans la partie de fixation (31), et/ou

dans lequel la part de contact de l'impédance (Z) dépend en outre de la résistance ohmique ($R_{23/40}$) de la couche barrière (23) dans la région de l'adhésif (40) introduit dans la région de la partie de fixation (31) et/ou de la résistance capacitive ($C_{23/40}$) de la couche barrière (23) dans la région de l'adhésif (40) introduit dans la partie de fixation (31).

**12.** Système de conducteur optique-électrique (100) selon l'une des revendications 10 ou 11, dans lequel l'impédance (Z) mesurable entre le manchon adaptateur (30) et la couche conductrice (22) du dispositif conducteur optique-électrique (1)

peut être mesurée entre le corps de manchon externe du manchon adaptateur (30) et une zone de la couche conductrice (22) dans une partie du dispositif conducteur optique-électrique (1) qui dépasse du manchon adaptateur (30) et est de préférence située côté extrémité, et/ou

peut être mesurée en présence d'une fréquence d'évaluation définie ou dans une plage de fréquences d'évaluation, la fréquence d'évaluation ou la plage de fréquences d'évaluation se situant de préférence dans la plage allant de 1 Hz à 10 GHz, notamment dans la plage allant de 10 Hz à 2,4 GHz, de manière plus avantageuse dans la plage allant de 100 Hz à 1 MHz, notamment dans la plage allant de 1 kHz à 100 kHz, et de manière particulièrement avantageuse dans la plage allant de 1 kHz à 10 kHz.

**13.** Système de conducteur optique-électrique (100) selon l'une des revendications précédentes, dans lequel le dispositif conducteur optique-électrique (1) comprend :

un système de couches fonctionnelles (20) se trouvant sur la couche de gainage extérieure (13) et comportant une zone de couche de base (21) constituée d'une couche individuelle ou d'une succession de couches, et la couche conductrice (22) électriquement conductrice, constituée d'une couche individuelle ou d'une succession de couches, la couche conductrice (22) étant appliquée sur la zone de couche de base (21), et
dans lequel le guide d'ondes optiques (10) comprend de préférence :

- un noyau (11) conducteur optique et
- de préférence une enveloppe (12) qui entoure le noyau (11) et est disposée entre le noyau (11) et la couche de gainage extérieure (13),
- et dans lequel le noyau (11) conducteur optique présente de préférence un indice de réfraction qui est supérieur à un indice de réfraction de la couche de gainage extérieure (13) et/ou supérieur à un indice de réfraction de l'enveloppe (12).

14. Système de conducteur optique-électrique (100) selon l'une des revendications précédentes, dans lequel la couche de gainage (13) organique comprend du polyamide, PA, polyimide, PI, ou du poly(méthacrylate de méthyle), PMMA, ou de la cire, des constituants semblables à la cire ou de l'alkylsilane.

15. Système de conducteur optique-électrique (100) selon l'une des revendications 13 à 14, présentant au moins l'une des caractéristiques suivantes a) à d) :

a) la zone de couche de base (21) comprenant :

- une couche appliquée sur la couche de gainage extérieure (13) et comportant un oxyde, en particulier $SiO_2$, $TiO_2$, $Al_2O_3$, $SnO_2$, $HfO_2$ ou un borure, carbure, nitrure, oxynitrure, carbonitrure ou un métal, en particulier Si, Ti, Mo ou Cr,
- ou une succession de telles couches appliquées sur la couche de gainage extérieure (13),

b) la zone de couche de base (21) comprenant ou étant constituée d'une couche superficielle de la couche de gainage extérieure (13), avec au moins une propriété de surface modifiée, en particulier une énergie superficielle accrue et/ou un nombre accru de radicaux d'oxygène, notamment fabriqués ou susceptibles d'être fabriqués à l'aide d'un processus chimique et/ou physique, en vue de la modification d'au moins une propriété de surface de la couche de gainage extérieure (13),
c) la zone de couche de base présentant une épaisseur comprise entre 5 nm et 3 000 nm, de préférence entre 5 nm et 1 000 nm, et de manière particulièrement avantageuse entre 10 nm et 100 nm, et/ou les différentes couches de la zone de couche de base présentant chacune une épaisseur comprise entre 5 nm et 1 000 nm, de préférence entre 10 nm et 100 nm,
d) sachant qu'il existe entre la zone de couche de base (21) et la couche conductrice (22) appliquée sur celle-ci, une adhérence qui est supérieure à une adhérence qui serait présente entre la couche de gainage extérieure (13) et une couche conductrice (22) identique appliquée sur celle-ci.

16. Système de conducteur optique-électrique (100) selon l'une des revendications 13 à 15, dans lequel au moins une des couches de la zone de couche de base (21) appliquées sur la couche de gainage extérieure (13) est conçue pour freiner la diffusion d'oxygène et/ou d'ions de solutions acides ou alcalines dans la couche conductrice (22).

17. Système de conducteur optique-électrique (100) selon l'une des revendications 13 à 16,

dans lequel la zone de couche de base (21) présente un coefficient de dilatation thermique qui se situe entre un coefficient de dilatation thermique de la couche de gainage (13) et un coefficient de dilatation thermique de la couche conductrice (22), et/ou
dans lequel la zone de couche de base comprend une succession de couches présentant chacune un coefficient de dilatation thermique qui augmente ou diminue en fonction de la succession des couches.

18. Système de conducteur optique-électrique (100) selon l'une des revendications précédentes, présentant au moins une des caractéristiques a) à c) :

a) la couche conductrice (22) comprenant :

- une couche appliquée sur la zone de couche de base (21) et comportant du titane, silicium, aluminium, or, argent, molybdène, tungstène ou zirconium, en particulier un alliage de l'une de ces matières avec Ni, Zn, Y, Sn, Ge,
- ou une succession de telles couches, appliquée sur la zone de couche de base (21),

b) la couche conductrice (22) présentant une épaisseur comprise entre 5 nm et 6 000 nm, de préférence entre 5 nm et 2 000 nm, et de manière particulièrement avantageuse entre 10 nm et 200 nm, et/ou des couches individuelles de la couche conductrice présentant chacune une épaisseur comprise entre 5 nm et 2 000 nm, de préférence entre 10 nm et 200 nm,
c) la couche conductrice (22) présentant une résistance de surface comprise entre 0,01 et 1 000 ohm/sqr, de préférence entre 0,01 et 100 ohm/sqr, et de manière particulièrement avantageuse entre 0,01 et 50 ohm/sqr.

19. Système de conducteur optique-électrique (100) selon l'une des revendications 13 à 18, dans lequel le système de couches fonctionnelles (20) comprend en outre :

- une couche barrière (23) appliquée sur la couche conductrice (22) et constituée d'une couche individuelle ou d'une succession de couches, la couche barrière (23) étant conçue pour freiner la diffusion d'oxygène et/ou d'ions de solutions acides ou alcalines dans la couche conductrice (22), et/ou
- une couche barrière (23) réalisée comme couche superficielle de la couche conductrice (22) avec au moins une propriété de matériau modifiée, et/ou réalisée comme couche d'oxydation, notamment naturelle, de la couche conductrice (22) et/ou comme film de contamination sur la couche conductrice (22).

20. Système de conducteur optique-électrique (100) selon la revendication 19, dans lequel au moins une des couches de la couche barrière (23) appliquées sur la couche conductrice (22) présente une dureté d'au moins 800 HV, de préférence d'au moins 1 200 HV, et de manière particulièrement avantageuse d'au moins 2 000 HV, selon la norme d'essai DIN EN ISO 14577-4:2007-8.

21. Système de conducteur optique-électrique (100) selon la revendication 19 ou 20, dans lequel la couche barrière (23) comprend :

- une couche appliquée sur la couche conductrice (22) et comportant un nitrure, notamment $Si_3N_4$, BN, AIN, TiN, AlSiN, SiON, SiAlON ou un alliage d'une de ces matières, ou comportant un oxyde, en particulier des oxydes de Si, Al, Ti, Zr, Zn, Sn, Ta, Nb, Y, $TiO_2$, $SiO_2$, ou un système ternaire constitué d'au moins une de ces matières ou comportant un carbure, borure, oxynitrure, carbonitrure,
- ou une succession de telles couches appliquées sur la couche conductrice (22).

22. Dispositif (200) pour détecter l'immersion d'un dispositif conducteur optique-électrique (1) dans un milieu conducteur (50), notamment dans le corps d'un animal ou d'un humain, comprenant :

un système de conducteur optique-électrique (100) selon l'une des revendications 1 à 21,
une unité d'évaluation (60) qui peut être reliée ou est reliée électriquement au manchon adaptateur (30), conducteur électrique au moins par portions, et au milieu conducteur (50), et qui est conçue pour déterminer une impédance ou une variation d'impédance entre le manchon adaptateur (30) et le milieu (50), lorsque le dispositif conducteur optique-électrique (1) est plongé ou est en cours d'immersion dans le milieu (50) conducteur, de telle sorte qu'une immersion du dispositif conducteur optique-électrique (1) dans le milieu (50) puisse être détectée et/ou une profondeur d'immersion du dispositif conducteur optique-électrique (1) dans le milieu (50) puisse être déterminée.

23. Procédé de fabrication d'un système de conducteur optique-électrique (100) selon l'une des revendications 1 à 21, comprenant :

la mise à disposition ou la fabrication du manchon adaptateur (30), conducteur électrique au moins par portions,
la mise à disposition ou la fabrication du dispositif conducteur optique-électrique (1),
l'introduction du dispositif conducteur optique-électrique (1) dans le manchon adaptateur (30), de manière à ce que le manchon adaptateur (30) saisisse mécaniquement le dispositif conducteur optique-électrique (1) et établisse un contact électrique avec la couche conductrice (22) de celui-ci,
le manchon adaptateur (30), mis à disposition ou fabriqué, comportant de préférence une partie de fixation (31) pour saisir mécaniquement le dispositif conducteur optique-électrique (1), et
un adhésif (40) étant de préférence introduit dans la partie de fixation (31) pour assurer une fixation supplémentaire du dispositif conducteur optique-électrique (1), et

l'introduction de l'adhésif conducteur électrique (41) dans la partie de contact (32), afin d'établir un contact électrique avec la couche conductrice (22) du dispositif conducteur optique-électrique (1).

**24.** Procédé selon la revendication 23, selon lequel la fabrication du dispositif conducteur optique-électrique (1) comprend :

- la mise à disposition du guide d'ondes optiques (10) avec la couche de gainage extérieure (13),
- l'application, sur la couche de gainage (13) du guide d'ondes optiques, d'un système de couches fonctionnelles (20), comprenant :

- la réalisation d'une zone de couche de base (21) constituée d'une couche individuelle ou d'une succession de couches,
- sur la zone de couche de base, application de la couche conductrice (22), conductrice de l'électricité, constituée d'une couche individuelle ou d'une succession de couches.

**25.** Procédé selon la revendication 24, selon lequel la réalisation de la zone de couche de base (21) comprend :

- le traitement préalable de la couche de gainage (13) du guide d'ondes optiques (10), notamment à l'aide de processus chimiques ou physiques, pour modifier les propriétés de surface de la couche de gainage (13), aux fins de réaliser une couche superficielle avec au moins une propriété de surface modifiée, notamment une couche superficielle avec une énergie superficielle accrue et/ou un nombre accru de radicaux d'oxygène, et/ou pour éliminer des résidus.

**26.** Procédé selon l'une des revendications 23 à 25,

selon lequel l'application d'au moins deux couches est réalisée sous vide et sans rupture du vide, et/ou
selon lequel l'application d'une ou plusieurs couches est réalisée avec ajout d'oxygène et d'argon, le rapport de l'oxygène au flux total d'oxygène et d'argon influençant le caractère métallique ou diélectrique de la couche concernée.

21 22 23

1

10, 10.1    20    11 12 13    13 20, 20.1 22.1  23.1

Figur 1

1

21 22 23

10    20    11    13    13 20

Figur 2

31 ——
31.1 ——
31.2, 40.2 ——

32 ——
32.1 ——
32.2, 41,2 ——
41 ——
41.1 ——

10 ——
10.1 ——

22 ——
22.1 ——
22.2 ——

23 ——
23.1 ——

50 ——

22.3 ——

100

200

30
40
40.1

60

1

Figur 3

Figur 4a

Figur 4b

**IN DER BESCHREIBUNG AUFGEFÜHRTE DOKUMENTE**

**In der Beschreibung aufgeführte Patentdokumente**

- DE 102012109088 A1
- DE 102006029203 A1
- US 5135295 A
- WO 2019057316 A1
- US 2019090998 A1
- WO 9422039 A1